# EUROPEAN PATENT SPECIFICATION

(11) **EP 3 378 484 B1**
(45) Date of publication and mention of the grant of the patent: **05.04.2023**
(21) Application number: 16864627.1
(22) Date of filing: 11.11.2016
(51) Int. Cl.: A61K 38/18, C12N 15/864, A61K 48/00, A61P 9/00

(54) **CCN5 PROTEIN FOR TREATMENT OF A HEART DISEASE ASSOCIATED WITH DUCHENNE MUSCULAR DYSTROPHY**
CCN5 PROTEIN ZUR BEHANDLUNG EINER HERZKRANKHEIT BEI DUCHENNE-MUSKELDYSTROPHIE
CCN5 POUR LE TRAITEMENT D'UNE MALADIE CARDIAQUE ASSOCIÉE À LA MYOPATHIE DE DUCHENNE

(30) Priority: 13.11.2015 KR 20150159888
(43) Date of publication of application: 26.09.2018
(73) Proprietor: BethphaGen Inc., Buk-gu, Gwangju 61005 (KR)
(72) Inventor: PARK, Woo Jin, Gwangju 61005 (KR); LEE, Min-Ah, Gwangju 61005 (KR)
(74) Representative: Dehns
(86) International application number: PCT/KR2016/013049
(87) International publication number: WO 2017/082701

(56) References cited:
- EP-A2- 2 556 839
- KR-A- 20110 105 957
- US-A1- 2008 207 489
- JEONG DONGTAK ET AL: "Matricellular Protein CCN5 Reverses Established Cardiac Fibrosis", JOURNAL OF THE AMERICAN COLLEGE OF CARDIOLOGY, ELSEVIER, NEW YORK, NY, US, vol. 67, no. 13, 28 March 2016 (2016-03-28), pages 1556-1568, XP029479730, ISSN: 0735-1097, DOI: 10.1016/J.JACC.2016.01.030
- JEONG et al.: "Abstract 20344: CCN5 Reverses Fibrosis and Cardiac Dysfunction Induced by Pressure Overload in Murine Models", Circulation, vol. 130, no. Supplement 2, 2014, XP009511189,
- YOON et al.: "The Opposing Effects ofCCN2 and CCN5 on the Development of Cardiac Hypertrophy and Fibrosis", Journal of Molecular and Cellular Cardiology, vol. 49, 2010, pages 294-303, XP027084519,
- XU et al.: "C(N5 Attenuates Profibrotic Phenotypes of Fibroblasts through the Smad6-CCN2 Pathway: Potential Role in Epidural Fibrosis", International Journal of Molecular Medicine, vol. 36, 21 April 2015 (2015-04-21), pages 123-129, XP055383587,
- ZHANG et al.: "CCN5 Overexpression Inhibits Profibrotic Phenotypes via the PI3K/Akt Signaling Pathway in Lung Fibroblasts Isolated from Patients with Idiopathic Pulmonary Fibrosis and in an In Vivo Model of Lung Fibrosis", International Journal of Molecular Medicine, vol. 33, 2014, pages 478-486, XP055383592,

## Description

### Technical Field

The present patent application claims the benefit of priority to Korean Patent Application No. 10-2015-0159888, filed on November 13, 2015 to Korean Intellectual Property Office.

The present invention relates to a treatments for heart diseases associated with Duchenne muscular dystrophy.

### Background Art

Myofibroblasts, which are the major contributor to cardiac fibrosis, are derived from differentiations of fibroblasts that are present in the cardiac tissue, vascular endothelial cells, and myeloid cells. Although source cells of myofibroblasts may be different, differentiated myofibroblasts are involved in overproduction of the extracellular matrix such as fibrotic collagen, *etc.* and the secretion thereof. The regression of contractile cells due to the expression of intracellular alpha smooth muscle actin (α-SMA) is defined as a decreased state compared to a time point where the degree of fibrosis occurs, and reversal or reversibility refers to a fully recovered state to a normal tissue structure.

Fibrotic diseases that account for approximately 45% of deaths caused in the Western society are a serious problem that can only be clinically diagnosed after the diseases have progressed very far (G. Garrison, S.K. Huang, et al., Am. J. Respir. Cell. Mol. Biol., 48:550558(2013); and Rosenbloom J., et al. Biochim. Biophys. Acta., 1832(7):1088-1103(2013)). Along with the limitation of preventive treatments, fibrotic diseases belong to a serious clinical field in which there is no effective treatment means for directly treating progressive or pre-existing fibrosis until the present (Rosenbloom J, Mendoza FA, et al., Biochim. Biophys. Acta, 1832(7):1088-1103(2013)). Although the causes of the fibrotic diseases for inducing fibrosis and the forms of diseases that occur may vary, it was found that myofibroblasts (MyoFB) in the middle of the mechanism of fibrosis formation, that is, activated fibroblasts, act as pathologically activated cells, thereby controlling the progression of all the fibrotic diseases (Dufferield J.S., et al., Annu. Rev. Pathol. Mech. Dis., 8:241-276(2013)).

Particularly, the heart is a representative organ in the human body in which structural remodeling of the cardiac muscular tissue due to fibrosis directly affects exercise function of the heart. The heart is a muscle tissue that is composed of various cells such as cardiomyocytes that play a pivotal role in exercise function, fibroblasts, and endothelial cells that are related to the blood vessel, *etc.* Although most of the volume of the cardiac muscles is composed of cardiomyocytes, fibroblasts account for 50% or more of the cells in the heart tissue. The functions of fibroblasts are to produce and secrete the extracellular matrix (ECM), which makes a precise structure that supports efficient contraction and relaxation of cardiomyocytes, and promotes transfer of appropriate force in the microenvironment inside the cardiac muscle tissue, transmission of electrical signal, intracellular communication, exchange of metabolites, *etc.* (F.G. Spinale, Physiol. Rev. 87:12851342(2007)). However, when fibrosis occurs due to an increase in the stress applied on the heart wall, the damage on the heart wall, diseases, etc., changes in the heart function occur due to stiffness of the heart tissue caused by overaccumulation of the fibrotic extracellular matrix (Schroer A.K., et al., J. Cell. Sci. 128(10):1865-1875(2015)). In cardiac fibrosis, myofibroblasts that are causative cells of fibrosis exhibit pathologically common environments such as cardiac damage, *etc.* (Kendall R.T., Feghali-Bostwick C.A., Front Pharmacol., 5: 123(2014)). Fibrosis-inducing growth factors and cytokines such as TGF-β, angiotensin-II (ANG-II), endothelin-I (ET-1), IL-6, connective tissue growth factor (CTGF), the extra domain A (EDA) of fibronectin, *etc.* that are secreted from damaged cardiac muscle cells and inflammatory immune cells are involved in the differentiation and activation of myofibroblasts (Frangogiannis N.G., Nat. Rev. Cardiol. 11(5):255-65(2014)). In the chronic disease state, myofibroblasts autonomously promote a vicious circle of fibrotic processes by continued proliferation and activation thereof. These factors include autocrine secretion of TGF-β1 and ANG-II, expression of the ANG-II type I receptor in fibroblasts by mechanical stimulation through stiffening of tissues due to overproduction of extracellular matrix, and provision of a new secretory cell function due to activation of genes that prevent apoptosis (Wynn T.A., Ramalingam T.R. Nat. Med. 18: 1028-1040 (2012)). The continuous proliferation and activation of myofibroblasts whose function of apoptosis has been lost lead to the production and accumulation of the extracellular matrix causing tissue remodeling, as well as the function of inflammatory cells. The secretion of oxygen free radical, lipid substances that conduct signal transduction, and cytokines such as TNF-α promotes, the inflow of inflammatory immune cells through the inflammation in damaged tissues and the secretion of chemokines such as MCP-1, *etc.,* thereby advancing the reactive fibrosis process (Van Linthout S., et al. , Cardiovasc. Res. 102(2):258-269(2014)). In the heart where prolonged activation of fibroblasts is induced, fibrotic tissues occur, thereby causing various pathologically adverse effects. 1) Surrounding cardiac muscle cells that are wrapped by fibrotic collagen are atrophied, thereby exist in various sizes, and reduce exercise load of muscle cells (Drakos S.G., et al. J. Am. Coll. Cardiol. 27;56(5):382-391(2010)). 2) The increase in passive tissue rigidity produces contractile fiber tissues by cross-linking of the secreted collagen type 1 and the gap junction of myofibroblasts, resulting in diastolic dysfunction of the heart (Lo B. et al. Hypertension 60:677683(2012)). 3) Perivascular fibrosis results in the apoptosis of cardiac muscle cells due to a decrease in normal energy metabolism because the abnormality in the contraction and relaxation of coronary artery vessels reduces oxygen supply in the cardiac muscle (Coen, M., Gabbiani, G. & Bochaton-Piallat, ML Arterioscler. Thromb. Vasc.Biol, 31:23912396(2011)). Therefore, when such fibrous remodeling is prolonged, a process of relaxation into systolic heart failure from diastolic heart failure is exhibited due to the contraction of cardiac muscle cells and the occurrence of apoptosis (Kamalov G., Zhao W., et al. J Cardiovasc. Pharmacol. 62(6):497-506(2013)).

The forms of fibrosis of cardiac tissues are classified into replacement fibrosis that forms scarred tissues to prevent rapid cardiac rupture when the necrosis of a large amount of cardiac muscle cells occurs such as myocardial infarction, produce extensive muscle cell necrosis, such as myocardial infarction, and interstitial and perivascular fibrosis that is reactive fibrosis gradually spreading due to various disease conditions (Bharath Ambale-Venkatesh and Joao A. C. Lima, Nat. Rev. Cardiol. 12: 18-29 (2015)). Heart diseases accompanied by cardiac fibrosis include 1) myocardial hypertrophy associated with genetic abnormality, 2) heart disease associated with chronic metabolic diseases such as hypertension, chronic kidney disease, diabetes, obesity, *etc.,* 3) heart valve disease, 4) heart disease associated with inflammatory diseases such as sarcoidosis, autoimmune myocarditis, and cardiac transplant-related rejection responses, *etc.,* 5) structural heart disease such as coronary dysfunction, aortic stenosis, nonischemic dilated cardiomyopathy, *etc.,* 6) infectious disease such as Chagas disease, viral myocarditis, *etc.,* 7) congenital heart disease such as cyanotic heart disease, tetralogy of Fallot, transposition of great arteries, single ventricle, *etc.,* 8) heart disease associated with genetic diseases such as Duchenne muscular dystrophy, Becker muscular dystrophy, Fabry disease, *etc.,* and 9) heart disease that occurs due to exposure of toxic chemicals such as smoking, alcohol intake, anticancer drugs, *etc.,* and in the natural aging process (Schelbert E.B., Fonarow G.C., J. Am. Coll. Cardiol. 63(21):2188-2198(2014); Collier P., et al., QJM. 105(8):721-724(2012); Maron B.J, Maron M.S., Lancet. 381(9862):242-255(2013); Kong P., Christia P., et al. Cell. Mol. Life Sci. 71(4):549-574(2014); and Mavrogeni S., Markousis-Mavrogenis G., et al. World J. Cardiol. (7):410-414(2015)). In addition, when fibrosis progresses regardless of the type of disease-causing factors, the heart shows a causal relationship between the rigidity of heart ventricles and, accordingly, heart failure such as systolic and diastolic cardiac dysfunction (Weber K.T., Sun Y., et al. Nat. Rev. Cardiol. 10 (1): 15-26 (2013)). Recently, heart failure has been clinically classified into two categories depending on abnormal cardiac function, which are normal ejection fraction whose direct cause is the fibrotic rigidity of the left ventricle heart failure with preserved ejection fraction (HFpEF) which exhibits and abnormal diastolic function, and heart failure with reduced ejection fraction (HFrEF) which exhibits reduced ejection fraction which is the cause of the loss of cardiac muscle cells, abnormality in the systolic function, and heart expansion (Burchfield J.S., Xie M., Hill J.A., et al. Circulation 128 (4): 388-400 (2013); and Butler J., Fonarow G.C., et al. JACC Heart Fail. 2(2): 97-112(2014)). Risk factors for HFpEF include aging, diabetes and metabolic diseases, cardiac hypertrophy, coronary artery disease, and hypertension, and these factors cause cardiac fibrosis and progress diastolic heart failure. Further, in the case of HFrEF, apoptosis such as myocardial infarction, *etc.* is the main cause, but in addition to alternative fibrosis, it was reported that an increase in the diffusion of interstitial fibrosis is proportional to an increase in severity of the disease, and acts as a factor for worsening the disease (Borlaug B.A., Nat. Rev. Cardiol. 11(9):507-515(2014)).

Preventive studies on cardiac fibrosis were reported in the heart failure model induced by pressure overload from transgenic mice with overexpressed hepatocyte growth factor (HGF) or transgenic mice in which endoglin gene which is a supporting factor of TGF-β is deficient. In each transgenic model mouse, endothelial-to-mesenchymal transition (EndoMT) of vascular endothelial cells to myofibroblasts was inhibited, which showed an effect of preventing the worsening of cardiac function of progressing to cardiac fibrosis and heart failure, but there was no verification of the therapeutic effect for fibrosis that was already generated (Okayama

K., Azuma J., et al. Hypertension 59(5):958-965(2012); and Kapur N.K., et al. Circulation 125(22):2728-2738(2012)).

Treatment of fibrosis requires a means of treatment to promote reversible return of the disease, such as removal of the excess-accumulated fibrotic tissue, restoration of the function of damaged tissue, *etc.* The regulation of senescence and death of myofibroblasts in the treatment of fibrotic diseases has been presented using the results of many studies with the possibility of effective therapeutic targets (Darby I.A., et al. Clin. Cosmet. Investig. Dermatol. 7:301-311(2014)). Recovery of the normal tissue structure and function in the healing process of *in vivo* tissue injury is started by activated fibroblasts becoming disappeared due to senescence and apoptosis. Dissolution and elimination of the accumulated extracellular fibrotic matrix are due to the secretion and cleaning action of collagenases in macrophages and fibroblasts, and after injury, recovery occurs (Wynn T.A, Ramalingam TR. Mechanisms of fibrosis: therapeutic translation for fibrotic disease. Nat Med. 18: 1028-40 (2012)). However, there is a report that the possibility of reversible treatment of fibrosis and recovery is related to the reversible recovery of symptoms of hepatic cirrhosis among patients who received antiretroviral treatment, and there is a report that the remodeling of the muscle structure is rarely reversed due to cardiac fibrosis in the process of using a left ventricular assist device for patients with heart failure and the function of the heart is improved (Sun M., Kisseleva T., Clin. Res. Hepatol. Gastroenterol. 39 Suppl. 1: S60-63(2015); and Jeff M. Berry, et al. Circ. Res. 109:407417(2011)). This fact is a new challenge to conventional knowledge that fibrosis cannot be reversibly treated up to date and presents a new treatment strategy for fibrosis. Therefore, the treatment of progressive and already-formed fibrosis is based on the mechanism of inhibiting proliferation of myofibroblasts which are pathogenic cells that are involved in the occurrence and progress of fibrosis and inducing apoptosis, through this treatment method, it is necessary to develop a therapeutic means that can regulate the formation and digestion of the fibrotic extracellular matrix and recover it to a normal tissue.

CCN5 is a substrate cell protein belonging to the CCN family, and six types of proteins are known in the CCN family, and it is reported that it plays various roles in regulating cell functions such as vascular disease, angiogenesis, carcinogenesis, induction of fibrosis diseases, cell differentiation, and survival (Perbal B. Lancet, 363:62-4(2004)). CCN5 has no C-terminal domain unlike other proteins in the CCN family and has other names such as WISP-2, HICP, Cop1, CTGF-L, *etc.* In addition, it consists of a single polypeptide chain of 250 amino acid sequences. CCN5 has a secretion induction sequence of 22 amino acids at its N-terminal, which is secreted extracellularly and functions as a signaling protein (Russo J.W., et al. J. Cell. Commun. Signal. 4(3):119-130(2010)).

Gene therapy is used in various fields for understanding the mechanism of diseases of cardiovascular diseases at genetic levels, discovering therapeutic genes, designing gene transfer vectors and packaging technology, and delivery technology for large animals in preclinical experiments and in clinical trials with patients (Mason D., et al. J. Control Release 215:101-111(2015)). In the case of a non-viral vector for gene vectors, clinical phase II results have been reported for improving the function of the heart of patients with myocardial infarction by a delivery method that directly injects a SDF-1 gene vector into the region around a wound of the heart. In addition, in the case of a viral vector, a method in which adenovirus (Ad) and adeno-associated virus (AAV) are directly injected into the myocardium by gene delivery therapy and a technique of delivery by passing the same locally to the coronary arteries and veins were used (Chung E.S., Miller L., et al. Eur. Heart J. 36(33):2228-2238(2015)). Clinical IIb of the gene therapy of AAV1-SERCA2a was finished, which improves the recovery of the contractive force and function of cardiac muscles for patients, and AAV9-S 100A1 and Ad5-Adenylyl-cyclase 6 are at clinical stages, and there was no serious side effect due to virus vectors (Rincon M.Y., et al. Cardiovasc. Res. 108(1):4-20(2015)). Therefore, through the studies provided herein, it was revealed that the CCN5 protein is effective in treating pre-existing cardiac fibrosis, which occurs due to various causes, through its selective apoptotic mechanism of myofibroblasts that are the pathogenic cells of fibrosis, by gene delivery method.

EP2556839A2 describes a pharmaceutical composition for preventing or treating a heart failure and a method for screening a therapeutic agent for preventing or treating a heart failure. The pharmaceutical composition comprises the CCN5 or CCN2ΔCT protein, or a genetic carrier comprising a nucleotide sequence encoding the CCN5 or the CCN2ΔCT protein.

Dongtak Jeong et al. (2016) "Matricellular Protein CCN5 Reverses Established Cardiac Fibrosis", Journal of the American College of Cardiology, vol. 16, no. 13, 1556-1568, discloses that CCN5 can reverse established cardiac fibrosis by inhibiting the generation of and enhancing apoptosis of myofibroblasts in the myocardium.

Dongtak Jeong et al. (2014) "Abstract 20344: CCN5 Reverses Fibrosis and Cardiac Dysfunction Induced by Pressure Overload in Murine Models", Circulation. Vol 130, A20344, showed that CCN5 evoked reversal of preformed cardiac fibrosis, which was accompanied by functional recovery of the failing hearts through the inhibition of transdifferentiation into myofibroblast and selective induction of apoptosis on myofibroblasts.

Yoon et al. (2010) "The Opposing Effects of CCN2 and CCN5 on the Development of Cardiac Hypertrophy and Fibrosis", Journal of Molecular and Cellular Cardiology, vol. 49 (2), 294-303, evaluated the role of CCN2 and CCN5 in the development of cardiac hypertrophy and fibrosis. Yoon *et al.* showed that CCN2 is pro-hypertrophic and -fibrotic, whereas CCN5 is anti-hypertrophic and -fibrotic. CCN5 lacking the CT domain acts as a dominant negative molecule.

### Disclosure of Invention

### Technical Problem

The inventors of the present invention have made intensive efforts to develop treatments for reversing cardiac fibrosis or a heart disease accompanied by cardiac fibrosis, more specifically a heart disease associated with Duchenne muscular dystrophy. As a result, it was found that the CCN5 gene or the CCN5 protein selectively kills myofibroblasts, markedly reduces cardiac fibrosis, and is capable of treating progressive or pre-existing cardiac fibrosis. It was also confirmed that there is a therapeutic effect on heart failure with reduced ejection fraction (HFrEF) or heart failure with preserved ejection fraction (HFpEF) accompanied by cardiac fibrosis.

An object of the present invention is to provide a pharmaceutical composition for use in treating a heart disease associated with Duchenne muscular dystrophy, wherein said composition comprises a CCN5 protein; or a gene carrier including a nucleotide sequence encoding the CCN5 protein, as an active ingredient and wherein said method comprises administering said composition to a subject in need thereof in an amount sufficient to induce myofibroblast-specific apoptosis.

Other objects and advantages of the present invention will become more apparent from the detailed description of the present invention, the appended claims, and the figures.

### Solution to Problem

According to one aspect of the present invention, the present invention provides a pharmaceutical composition for use in a method for treating a heart disease associated with Duchenne muscular dystrophy, wherein said composition comprises a CCN5 protein; or a gene carrier including a nucleotide sequence encoding a CCN5 protein, as an active ingredient and wherein said method comprises administering said composition to a subject in need thereof in an amount sufficient to induce myofibroblast-specific apoptosis .

As a result of intensive research efforts to develop a method for reversibly treating cardiac fibrosis or a heart disease accompanied by cardiac fibrosis, more specifically a heart disease associated with Duchenne muscular dystrophy, the present inventors have found that CCN5 gene or CCN5 protein selectively kills myofibroblasts to significantly reduce cardiac fibrosis, and it is possible to treat progressive or pre-existing cardiac fibrosis and have a therapeutic effect on heart failure with reduced ejection fraction (HFrEF) or diastolic heart failure with preserved ejection fraction (HFpEF).

The heart disease associated with Duchenne muscular dystrophy treatable in accordance with the invention may involve progressive cardiac fibrosis or pre-existing cardiac fibrosis. As described above, recently, there is a possibility that fibrosis can be reversibly treated, and as demonstrated in the example below, myofibroblasts are selectively killed by the pharmaceutical composition of the present invention, and accordingly, pre-existing fibrosis was recovered thereby, thus the therapeutic effect for progressive cardiac fibrosis or pre-existing cardiac fibrosis was confirmed. Therefore, an important characteristic of the present invention is that the pharmaceutical composition of the present invention induces myofibroblast-specific apoptosis.

According to one embodiment of the present invention, the CCN5 protein of use in the present invention includes an amino acid sequence represented by SEQ ID NO: 1.

According to one embodiment of the present invention, the nucleotide sequence encoding the CCN5 protein of use in the present invention consists of a nucleotide sequence represented by SEQ ID NO: 2.

A gene carrier including a nucleotide sequence encoding the CCN5 protein, which is an active ingredient of the pharmaceutical composition of the present invention, is used in a gene delivery system that transfers the CCN5 gene to the heart. The term "gene transfer" as used herein means that a gene is carried into a cell and has the same meaning as intracellular transduction of a gene. At the tissue level, the term "gene delivery" has the same meaning as "spread of a gene". Therefore, the gene delivery system of use in the present invention can be described as a gene transduction system and a gene spreading system.

To prepare the gene delivery system of use in the present invention, the nucleotide sequence encoding the CCN5 protein (e.g., the CCN5 protein gene) can be present in an appropriate expression construct. In the above expression construct, the CCN5 protein gene can be operatively connected to a promoter. As used herein, the term "operably linked" refers to a functional association between a nucleic acid expression control sequence (e.g., promoter, signal sequence, or an array of the binding site of a transcriptional regulator) and another nucleic acid sequence, thereby the control sequence regulates transcription and/or translation of the other nucleic acid sequence. The promoter linked to the CCN5 protein gene sequence, according to one embodiment of the present invention, functions in an animal cell and, according to another embodiment, functions in a mammalian cell, to regulate the transcription of the CCN5 protein gene, and it includes a promoter derived from a mammalian virus and a promoter derived from the genome of mammalian cells, and may include, for example, a cytomegalovirus (CMV) promoter, an adenovirus late promoter, a vaccinia virus 7.5K promoter, an SV40 promoter, a tk promoter of HSV, an RSV promoter, an EF1 alpha promoter, a metallothionein promoter, a beta-actin promoter, a promoter of the human IL-2 gene, a promoter of the human IFN gene, a promoter of the human IL-4 gene, a promoter of the human lymphotoxin gene, and a promoter of the human GM-CSF gene, but not limited thereto. According to a particular embodiment of the invention, the promoter is a CMV promoter.

Although the gene carrier of use in the present invention may be prepared in various forms, it can be prepared in the form of a virus carrier or a non-virus carrier, and more specifically, (i) a naked recombinant DNA molecule, (ii) a plasmid, (iii) a viral vector, and (iv) a liposome or niosome encapsulating the naked recombinant DNA molecule or plasmid.

The nucleotide sequence encoding the CCN5 protein of use in the present invention can be applied to all gene delivery systems utilized for conventional gene therapy, preferably, to plasmids, adenoviruses (Lockett LJ, et al., Clin. Cancer Re. 3:2075-2080(1997)), adeno-associated viruses (AAV, Lashford L. S., et al., Gene Therapy Technologies, Application and Regulations Ed. A. Meager, 1999), retroviruses (Gunzburg W.H., et al., Retroviral vectors. Gene Therapy Technologies, Application and Regulations Ed. A. Meager, 1999), lentiviruses (Wang G. et al., J. Clin. Invest. 104(11): R55-62(1999)), herpes simplex viruses (Chamber R., et al., Proc. Natl. Acad. Sci. USA 92:1411-1415(1995)), vaccinia viruses (Puhlmann M. et al., Human Gene Therapy 10:649-657(1999)), liposomes (Methods in Molecular Biology, Vol 199, S.C. Basu and M. Basu (Eds), Humana Press 2002), or niosomes.

### Adenovirus

Adenovirus is widely used as a gene transfer vector due to its moderate genome size, convenience of operation, high titer, wide range of target cells, and superior infectivity. Both ends of the genome contain an inverted terminal repeat (ITR) of 100 to 200 bp, which is a cis element essential for DNA replication and packaging. The E1 region of the genome (E1A and E1B) encodes a protein involved in viral DNA replication.

Among the adenoviral vectors currently developed, replication-incompetent adenovirus lacking the E1 region is widely used. Meanwhile, the E3 region is deleted from the conventional adenoviral vector and provides a site in which foreign genes are inserted (Thimmappaya, B. et al., Cell, 31:543-551(1982); and Riordan, J.R. et al., Science, 245:1066-1073(1989)). Therefore, it is preferable that the CCN5 protein gene of use in the present invention is inserted into the deleted E1 region (E1A region and/or E1B region, preferably E1B region) or E3 region, more preferably into the deleted E3 region. Meanwhile, the target nucleotide sequence to be intracellularly delivered is inserted in the deleted E1 region (E1A region and/or E1B region, preferably E1B region) or E3 region, preferably E3 region. In addition, it can also be expressed by a bicistronic expression system in which "promoter-target nucleotide sequence-poly A sequence-IRES-CCN5 protein gene" is connected by the internal ribosome entry site (IRES).

In addition, since adenovirus can pack up to about 105% of the wild-type genome, about 2 kb can be additionally packed (Ghosh-Choudhury et al., EMBO J., 6:1733-1739(1987)). Therefore, the foreign sequences described above, which are inserted into the adenovirus, can be further linked to the adenovirus genome.

Adenoviruses have 42 different serotypes and A-F subgroups. Among them, the adenovirus type 5 belonging to the subgroup C is the most preferred starting material for obtaining the adenoviral vector of use in the present invention. The biochemical and genetic information of the adenovirus type 5 is well known in the art.

Foreign genes delivered by adenovirus are replicated in the same way as episomes, and the genetic toxicity against host cells is very low. Therefore, it is judged that gene therapy using the adenovirus gene delivery system of use in the present invention is very safe.

### Retrovirus

Retroviruses are used as gene transfer vectors because these viruses can insert their own genes into the genome of the host, carry a large quantity of foreign genetic material, and have a broad spectrum of cells that can be infected.

To construct a retroviral vector, the CCN5 protein gene and the target nucleotide sequence to be transferred are inserted into the retroviral genome instead of the retroviral sequence to produce a replication-incompetent virus. In order to generate virions, packaging cell lines are constructed, which contain gag, pol, and env genes, but not long terminal repeats (LTR) and ψ sequences (Mann et al., Cell, 33: 153-159 (1983)). When transferring the recombinant plasmid including the CCN5 protein gene, the target nucleotide sequence to be delivered, the LTR and the ψ sequence into the cell line, the ψ sequence allows production of the RNA transcriptome of the recombinant plasmid, the transcriptome is packaged in a virus and the virus is discharged into the medium (Nicholas and Rubinstein "Retroviral vectors", In: Vector: A survey of molecular cloning vectors and their uses, Rodriguez and Denhardt (eds.), Stoneham L Butterworth, 494-513 (1988)). The medium containing the recombinant retrovirus is collected, concentrated, and used as a gene delivery system.

Gene transfer using the second-generation retroviral vector was reported. Kasahara, et al. (Science, 266: 1373-1376 (1994)) produced a variant of Mohrluny murine leukemia virus, where an erythropoietin (EPO) sequence was inserted at the envelope site to produce a chimeric protein. The gene delivery system of use in the present invention can also be produced based on such a second-generation retroviral vector construction strategy.

### AAV vector

Adeno-associated virus (AAV) is suitable for the gene delivery system of use in the present invention because it can infect non-dividing cells and has the ability to infect various types of cells. A detailed description of the preparation and use of AAV vectors is disclosed in detail in U.S. Patent Nos. 5,139,941 and 4,797,368.

Studies on AAV as a gene delivery system are described in LaFace, et al., Viology, 162: 483-486 (1988), Zhou, et al., Exp. Hematol. (NY), 21: 928-933 (1993), Walsh, et al., J. Clin. Invest., 94: 1440-1448 (1994) and Flotte, et al., Gene Therapy, 2:29-37(1995). Recently, clinical phase 2 is carried out for the AAV vector as a treatment for heart failure.

Typically, the AAV virus is a plasmid including a target gene sequence (CCN5 protein gene and the target nucleotide sequence to be delivered), in which two AAV terminal repeats are arranged side by side (McLaughlin, et al., J. Virol. And Samulski, et al., J. Virol., 63: 3822-3828 (1989)) and expression plasmids including wild type AAV coding sequences without terminal repeats (McCarty, et al. J. Virol., 65:2936-2945(1991)).

The AAV virus has nine different serotypes (AAV1, AAV2, AAV3, AAV4, AAV5, AAV6, AAV7, AAV8, and AAV9). Among these, AAV1, AAV6, AAV8, or AAV9 is the most preferred starting material for obtaining the adeno-associated virus vector of use in the present invention.

### Other viral vectors

Other viral vectors can also be used as the gene delivery system of use in the present invention. Vaccinia virus (Puhlmann M, et al., Human gene therapy 10: 649-657 (1999); Ridgeway, "Mammalian expression vectors," In: Vectors: A survey of molecular cloning vectors and their uses. Rodriguez and Denhardt, eds. Stoneham: Butterworth, 467-492 (1988); Baichwal and Sugden, "Vectors for gene transfer derived from animal DNA viruses: Transient and stable expression of transferred genes," In: Kucherlapati R, ed. Gene transfer. New York: Plenum Press, 117-148 (1986) and Coupar, et al., Gene, 68: 1-10(1988)). Vectors derived from lentivirus (Wang G., et al., J. Clin. Invest. 104 (11): R55-62(1999)) or herpes simplex virus (Chamber R., et al., Proc. Natl. Acad. Sci USA 92:1411-1415(1995)) can also be used as a delivery system capable of carrying the CCN5 protein gene and the nucleotide sequence of interest to be delivered into the cell.

### Liposome

Liposomes are automatically generated by phospholipids dispersed in water. Examples of successfully delivering foreign DNA molecules to liposomes are described in Nicolau and Sene, Biochim. Biophys. Acta, 721: 185-190 (1982) and Nicolau, et al., Methods Enzymol., 149:157-176(1987). Meanwhile, lipofectamine (Gibco BRL) is the most widely used reagent for transformation of animal cells using liposomes. Liposomes encapsulating the CCN5 protein gene and the target nucleotide sequence to be delivered interact with cells and deliver the CCN5 protein gene and the target nucleotide sequence to be delivered into the cell via mechanisms such as endocytosis, adsorption to the cell surface, fusion with plasma cell membranes, *etc.*

In accordance with the present invention, when a gene carrier is prepared based on a viral vector, the method for injecting the pharmaceutical composition of the present invention is carried out according to virus infection methods known in the art. Infection of host cells using viral vectors is described in the referenced documents mentioned above.

In accordance with the present invention, when the gene delivery system is a naked recombinant DNA molecule or a plasmid, the microinjection method (Capecchi, M.R., Cell, 22:479(1980); and Harland and Weintraub, J. Cell Biol. 101:1094-1099(1985)), the calcium phosphate precipitation method (Graham, F.L., et al., Virology, 52:456(1973); and Chen and Okayama, Mol. Cell. Biolo. 7:2745-2752(1987)), the electroporation method (Neumann, E., et al., EMBO J., 1:841(1982); and Tur-Kaspa, et al., Mol. Cell. Biol., 6:716-718(1986)), the liposome-mediated transfection method (Wong, T.K., et al., Gene, 10:87(1980); Nicolau and Sene, Biochim. Biophys. Acta, 721:185-190(1982); and Nicolau, et al., Methods Enzymol., 149:157-176(1987)), DEAE-Dextran treatment method (Gopal, Mol. Cell Biol., 5:1188-1190(1985)), and gene bombardment (Yang, et al., Proc. Natl. Acad. Sci., 87:9568-9572(1990)) can be used to insert genes into cells.

Detailed description of the gene carriers that can be used in the present invention is disclosed in detail in U.S. Patent Publication No. 2013/0287739.

According to one embodiment of the present invention, the gene carrier of use in the present invention is selected from the group consisting of a plasmid, an adenovirus, an adeno-associated virus, a retrovirus, a lentivirus, a herpes simplex virus, vaccinia virus, a liposome, and a niosome. According to another embodiment of the present invention, the gene carrier of use in the present invention is an adeno-associated virus, and according to still another embodiment of the present invention, the adeno-associated virus of use in the present invention is selected from the group consisting of the adeno-associated virus serotype 1, 6, 8, and 9. According to a particular embodiment of the present invention, the gene carrier of use in the present invention is the adeno-associated virus serotype 9.

According to the present invention, the pharmaceutical composition of the present invention induces myofibroblast-specific apoptosis and may inhibit the differentiation of fibroblasts into myofibroblasts and is thereby effective in treating the heart disease associated with Duchenne muscular dystrophy.

As demonstrated in the example below, the pharmaceutical composition of the present invention expresses CCN5 in a diseased heart through the gene therapy of AAV9-CCN5 in a pressure overload model, which is a model of systolic heart failure, a model of muscle-regressive diastolic heart disease, and a model of diastolic Aortic banding-Ischemia-reperfusion-Debanding (AID) model, in order to reversibly treat cardiac fibrosis to prevent the reduction systolic and diastolic cardiac function or treat the same. In addition, since the pharmaceutical composition of the present invention also increases the SERCA2a protein responsible for the pump function of blood circulation by increasing the cardiac systolic force, the direct therapeutic effect on HFrEF can also be predicted, and therefore, and, thereby the therapeutic effect can also be predicted when symptoms of HFrEF and HFpEF coexist.

Cardiac fibrosis diseases include a heart disease selected from the group consisting of hypertrophic cardiomyopathy; a heart disease due to chronic metabolic diseases such as hypertension, chronic kidney disease, diabetes, and obesity; valvular heart disease; inflammatory heart disease such as sarcoidosis, autoimmune myocarditis, a rejection in cardiac transplantation, *etc.;* inflammatory heart disease such as coronary artery dysfunction, aortic stenosis, nonischemic dilated cardiomyopathy, *etc.;* structural heart disease; a heart disease due to contagious pathogenic infection such as Chagas disease and viral myocarditis, *etc.;* congenital heart disease such as cyanotic heart disease, tetralogy of Fallot, transposition of great arteries, single ventricle, *etc.;* a heart disease due to hereditary anomaly such as Duchenne muscular dystrophy, Becker muscular dystrophy, Fabry disease, *etc.;* a heart disease due to smoking, alcohol intake, or exposure to toxic drugs (e.g. anticancer drugs); and heart disease due to aging..

The pharmaceutical composition of the present invention may include a pharmaceutically acceptable carrier. The pharmaceutically acceptable carrier included in the pharmaceutical composition of the present invention is what is conventionally used for formulation, and the examples thereof include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil, *etc*., but the present invention is not limited thereto. The pharmaceutical composition of the present invention may further include a lubricant, a wetting agent, a sweetener, a flavoring agent, an emulsifying agent, a suspending agent, a preservative, *etc.* in addition to the above components. Suitable pharmaceutically acceptable carriers and formulations are described in detail in Remington's Pharmaceutical Sciences (19th ed., 1995). The injection amount of the pharmaceutical composition of the present invention is desirably determined in consideration of the age, sex, and condition of the patient, the degree of absorption of active ingredients in the body, the inactivation rate, and the drug to be used in combination with the drug currently taken by the patient, and can be injected at a dose of 0.0001 mg/kg (body weight) to 100 mg/kg (body weight) based on the nucleotide encoding the CCN5 protein gene.

The pharmaceutical composition of the present invention can be administered either orally or parenterally, and the parenteral administration includes intravenous injection, subcutaneous injection, intramuscular injection, intraperitoneal injection, percutaneous injection, direct injection into tissues, *etc.*

The dosage form of the pharmaceutical composition may differ depending on the use method, but it can be prepared as plasters, granules, powders, syrups, solutions, fluidextracts, emulsions, suspensions, infusions, tablets, injections, capsules, and pills, *etc.*

The pharmaceutical composition of the present invention may be formulated by using a pharmaceutically acceptable carrier and/or excipient(s) according to a method which can be easily carried out by a person having ordinary knowledge in the technical field to which the invention belongs, and it may be prepared by infusing in a unit volume form or in a multi-volume container and may further include a dispersant or a stabilizer.

An active ingredient used in the pharmaceutical composition of the present invention is not only the gene carrier itself including the above CCN5 protein or the nucleotide sequence encoding the same, but also a pharmaceutically acceptable salt, hydrate, or solvate.

As used herein, the term "pharmaceutically acceptable salt" refers to a salt of an active ingredient of use in the present invention which has the desired pharmacological effect, that is, induces myofibroblast-specific apoptosis, and optionally inhibits the differentiation of fibroblasts and has an activity of selectively killing fibroblasts. These salts are formed by using inorganic acids such as hydrochloride, hydrobromide and hydroiodide and organic acids such as acetate, adipate, alginate, aspartate, benzoate, benzenesulfonate, p-toluenesulfonate, bisulfate, sulfamate, sulfate, naphthylate, butyrate, citrate, camphorate, camphorsulfonate, cyclopentane propionate, digluconate, dodecylsulfate, ethanesulfonate, fumarate, glucoheptanoate, glycerophosphate, hemisulfate, heptanoate, hexanoate, 2-hydroxyethane sulfate, lactate, maleate, methanesulfonate, 2-naphthalenesulfonate, nicotinate, oxalate, tosylate, and undecanoate. As used herein, the term "pharmaceutically acceptable hydrate" refers to a hydrate of CCN5 having the desired pharmacological effect. As used herein, the term "pharmaceutically acceptable solvate" refers to a solvate of an active ingredient of use in the present invention having the desired pharmacological effect. The above-mentioned hydrate and solvate can also be prepared using the above-mentioned acids.

Also described herein is a method for screening for a pharmaceutical composition for treating cardiac fibrosis or a heart disease accompanied by cardiac fibrosis, including the steps of: (a) treating a test substance to cells including CCN5 protein or CCN5 gene; (b) analyzing the expression of the CCN5 protein or CCN5 gene.

According to the above method First, a sample to be analyzed is brought into contact with cells including CCN5 protein or CCN5 gene. In certain instances the cell including the CCN5 protein or CCN5 gene is a cell derived from a heart. The term "test substance", which is used while referring to the above screening method, refers to an unknown substance used in screening to check whether it affects the expression level of the CCN5 protein gene or the amount of the CCN5 protein. The test substance includes, but is not limited to, chemical substances, nucleotides, anti-sense RNA, siRNA (small interference RNA), and natural product extracts.

Subsequently, the expression level of the CCN5 gene or the amount of the CCN5 protein is measured in the cells treated with the test substance. As a result of the measurement, when it is determined that the expression level of the CCN5 gene or the amount of CCN5 protein is up-regulated, the test substance can be designated as a therapeutic agent for cardiac fibrosis; or that for a heart disease accompanied by cardiac fibrosis.

Measurement of changes in the expression level of the CCN5 gene can be carried out via various methods known in the art. For example, RT-PCR (Sambrook, et al., Molecular Cloning. A Laboratory Manual, 3rd ed. Cold Spring Harbor Press (2001)), northern blotting (Peter B. Kaufman, et al., Molecular and Cellular Methods in Biology and Medicine, 102-108, CRC Press), hybridization reaction using cDNA microarray (Sambrook, et al., Molecular Cloning. A Laboratory Manual, 3rd ed. Cold Spring Harbor Press (2001)) or *in situ* hybridization reaction (Sambrook, et al., Molecular Cloning. A Laboratory Manual, 3rd ed. Cold Spring Harbor Press (2001)), *etc.*

When carrying out on the basis of the RT-PCR protocol, first, total RNA from the cells treated with the test substance is separated, and then the first strand cDNA is prepared using oligo dT primer and reverse transcriptase. Subsequently, a PCR reaction is performed using the CCN5 protein gene-specific primer set using the first strand cDNA as a template. Therefore, PCR amplification products are electrophoresed, and the formed bands are analyzed to measure changes in the expression level of the CCN5 protein gene.

Also described herein is a method for treating cardiac fibrosis or a heart disease accompanied by cardiac fibrosis by administering to a subject a pharmaceutical composition including (a) CCN5 protein; or (b) a gene carrier including a nucleotide sequence encoding CCN5 protein, as an active ingredient.

The above treatment method may use the above-described pharmaceutical composition of the present invention, and for the contents common in relation to the above-mentioned pharmaceutical composition of the present invention, the description thereof is omitted in order to avoid excessive complexity.

### Advantageous Effects of Invention

The features and advantages of the present invention are summarized as follows:
(A) The present invention provides a pharmaceutical composition for the treatment of a heart disease associated with Duchenne muscular dystrophy.
(B) Since the pharmaceutical composition of the present invention has effects of dissolving a fibrotic substrate by selectively killing myofibroblasts in cardiac fibrosis and of recovering the same to a normal tissue, it can be effectively used as a pharmaceutical composition for treating a heart disease associated with Duchenne muscular dystrophy which has been regarded as irreversible disease progression and for which a therapeutic drug has not been developed so far.

### Brief Description of Drawings

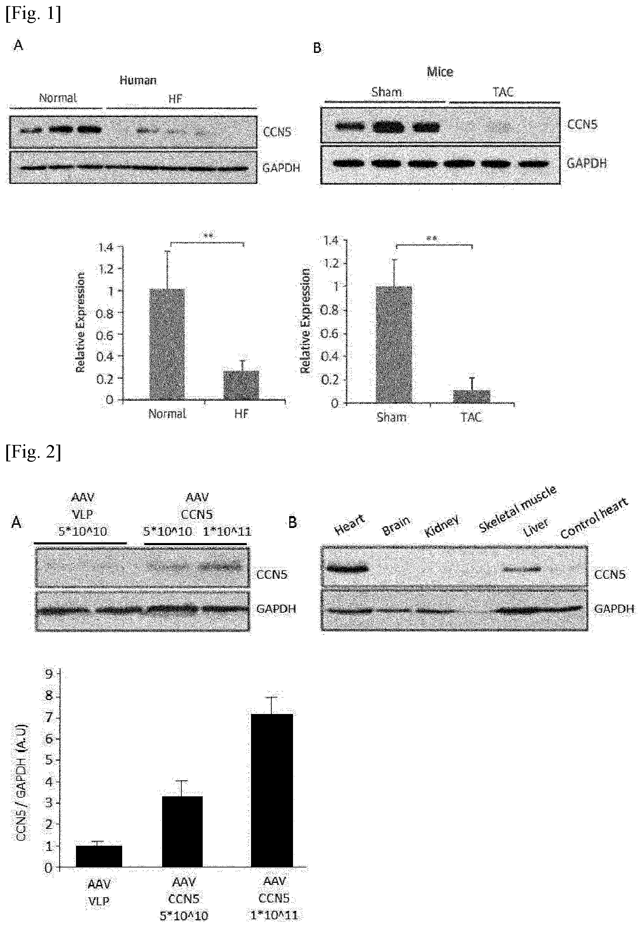

Fig. 1 is the result of determining changes in the expression level of CCN5 protein in the cardiac tissue of heart failure from (A) humans and (B) mice with anti-CCN5 and anti-GAPDH antibodies (** P < 0.01).
Fig. 2 shows the amount of expression of CCN5 protein in cardiac tissue 4 weeks after injection of AAV9-CCN5 into the tail vein of experimental rats (n = 6, * P < 0.05).

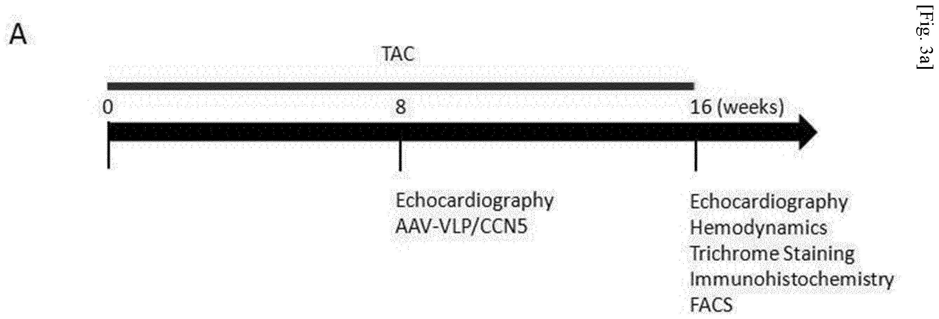

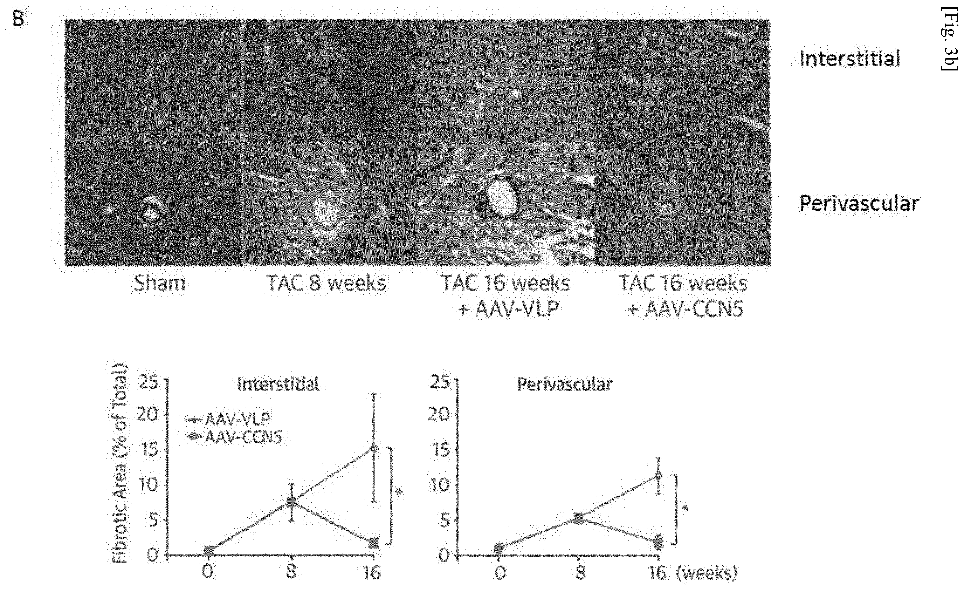

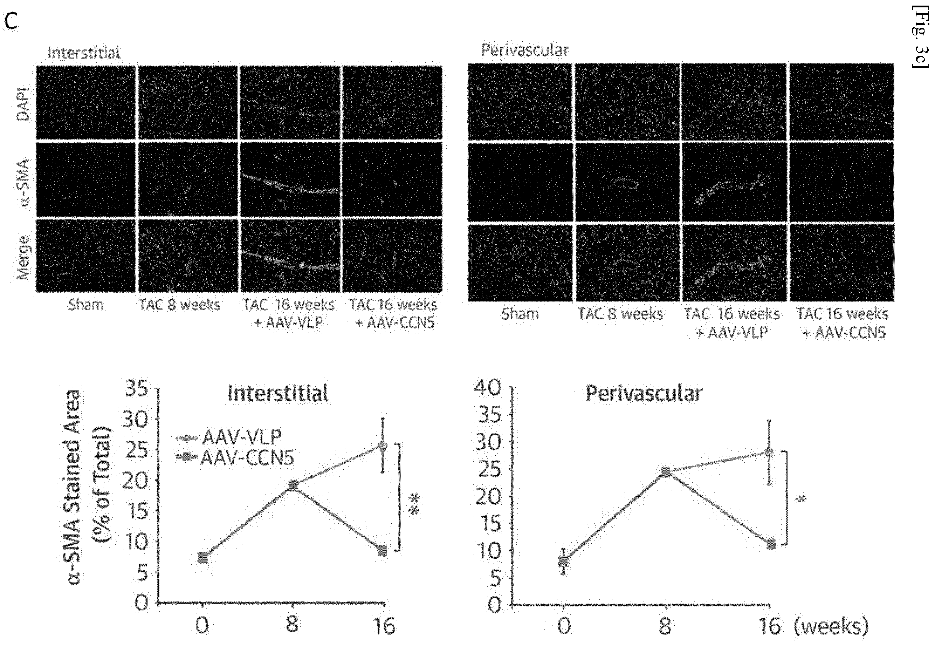

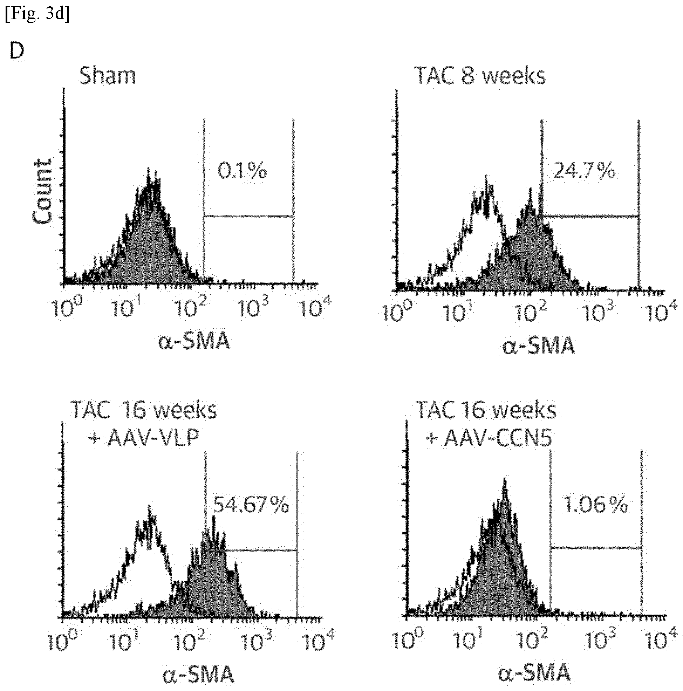

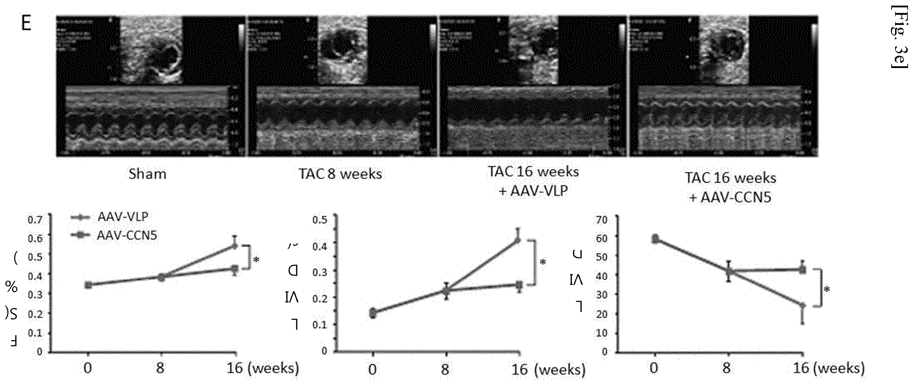

Figs. 3a-3e show the reversible therapeutic effect and cardiac function protective effect of AAV9-CCN5 for pre-existing cardiac fibrosis in pressure overload model experimental rats. Fig. 3a shows the experimental schedule of pressure overload model induction and AAV-CCN5 gene therapy injection to determine the reversible therapeutic effect. The upper part of Fig. 3b shows the degree of fibrosis of a cardiac tissue using the Masson-Trichrome staining method. The upper part is interstitial tissue fibrosis, the lower part is perivascular tissue fibrosis, and the percentage of fibrosis in each tissue is indicated by dots. Fig. 3c is the result of staining the cross section of the cardiac tissue with anti-α-SMA (myofibroblast target protein) antibody using fluorescent staining. The left representative image shows an interstitial tissue, and the right representative image shows a perivascular tissue. The lower part is a graph showing the degree of α-SMA expressing cells. Fig. 3d is the result of measurement by flow cytometry (FACS, n = 3, ^{∗} P < 0.05, ^{∗∗} P < 0.01). Fig. 3e measured the fractional shortening (FS) and the left ventricular internal dimension at systole (LVIDs) of the heart, and the left ventricular internal dimension at diastole (LVIDd) (n = 16, ^{∗} P < 0.05).

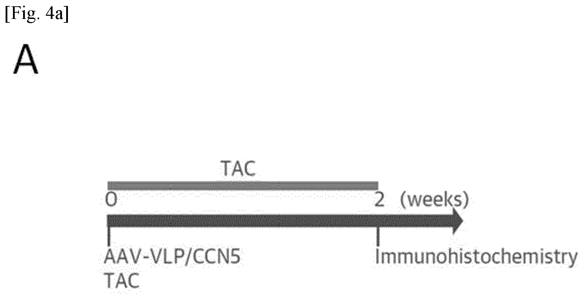

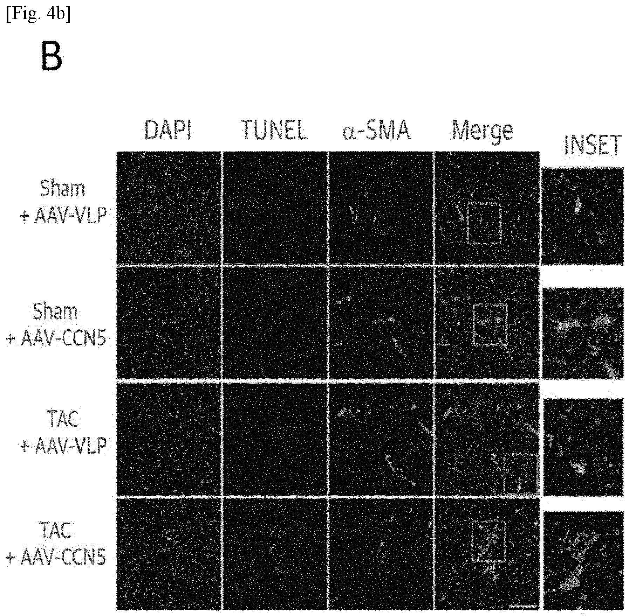

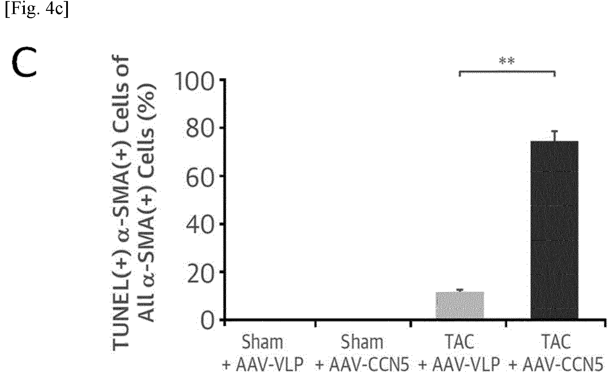

Figs. 4a-4c show that AAV9-CCN5 and CCN5 proteins induce apoptosis of myofibroblasts *in vivo* and at the cellular level. Fig. 4a shows an experimental schedule in a pressure overload rat model, and Fig. 4b shows a result of staining cardiac tissue slices simultaneously with TUNEL staining (red) and anti-α-SMA antibody (green) which is a specific protein of fibroblasts. Fig. 4c also quantitatively shows the degree of apoptosis of myofibroblasts (n = 3, ^{∗∗} P < 0.01) for each experimental group in Fig. 4b.

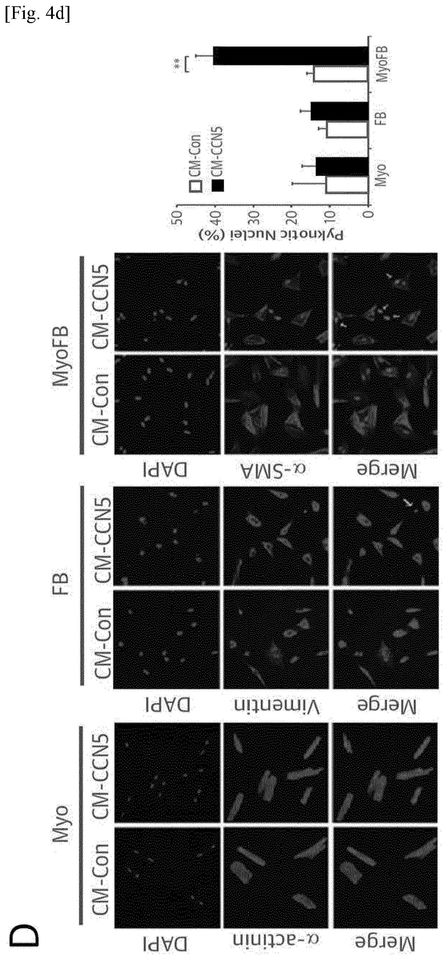

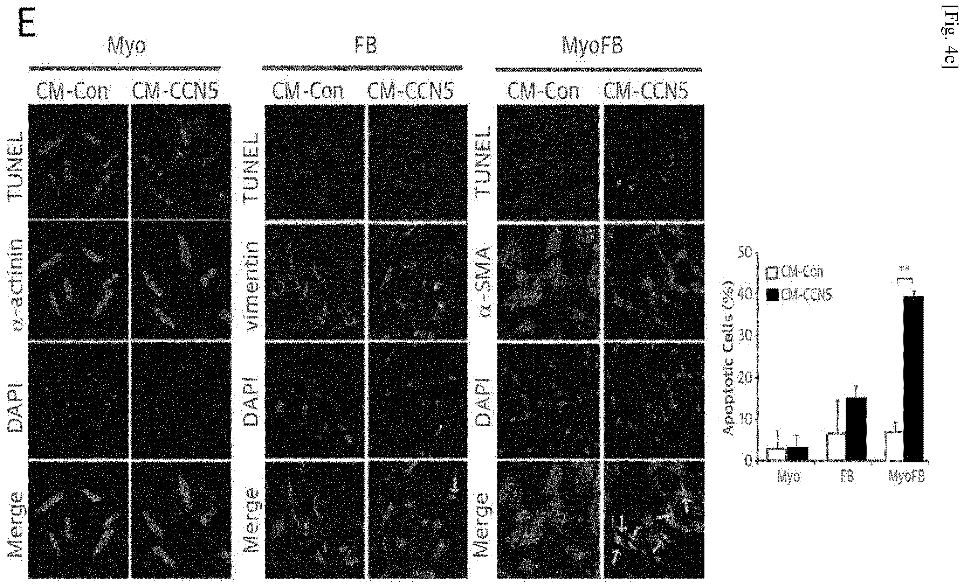

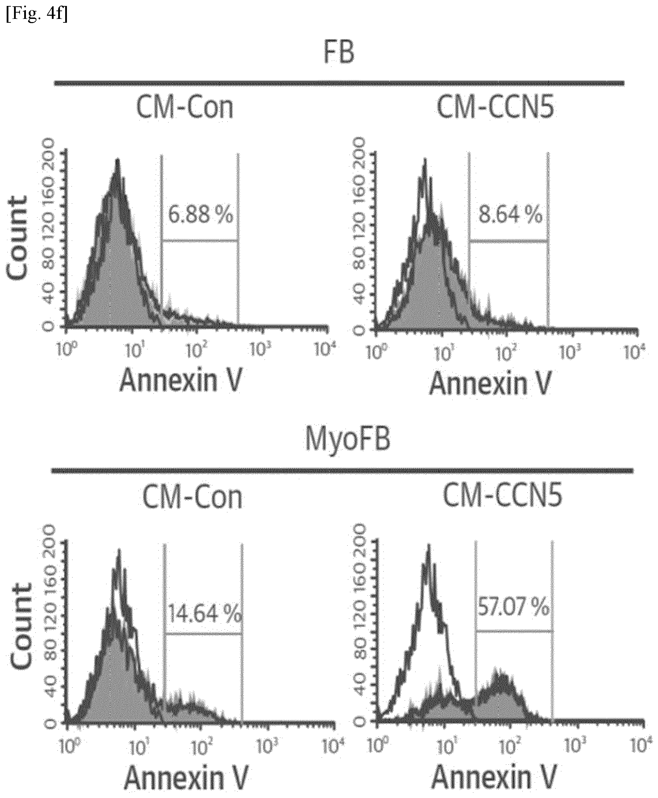

Figs. 4d-4f show the result of experiments after culturing cardiomyocytes (Myo), fibroblasts (FB), and myofibroblasts (MyoFB) for 48 hours in a control cell culture medium and a cell culture medium including CCN5 protein. Fig. 4d is the result of staining with DAPI, wherein the arrow indicates pyknotic nuclei, and the right figure shows the number of pyknotic nuclei in a graph. Fig. 4e is the result of staining the cells cultured under the same condition with TUNEL staining, wherein the arrow indicates nuclei showing TUNEL, and the right figure is the number of nuclei showing TUNEL fluorescence shown in a graph (n = 3, ^{∗∗} P < 0.01). Fig. 4f shows the result of analysis by fluorescence-activated cell sorting (FACS) after culturing fibroblasts and myofibroblasts in a control medium (CM-Con) and a medium (CM-CCN5) including CCN5 protein for 48 hours (n = 3, ^{∗∗} P < 0.01).

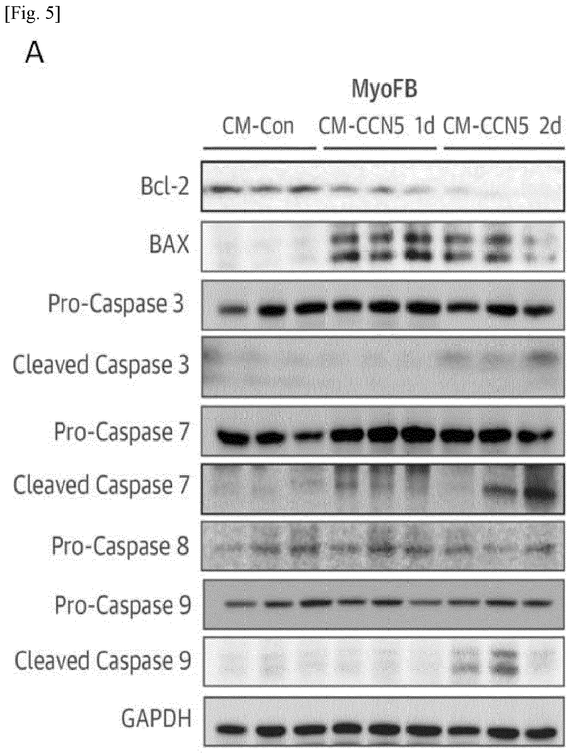

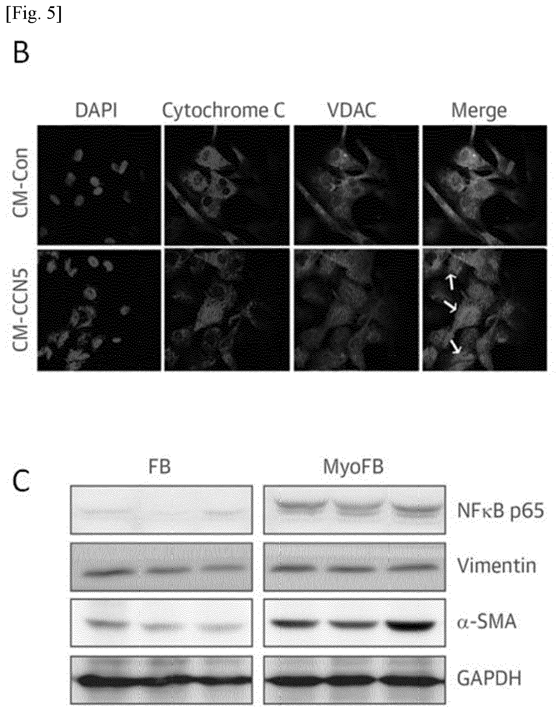

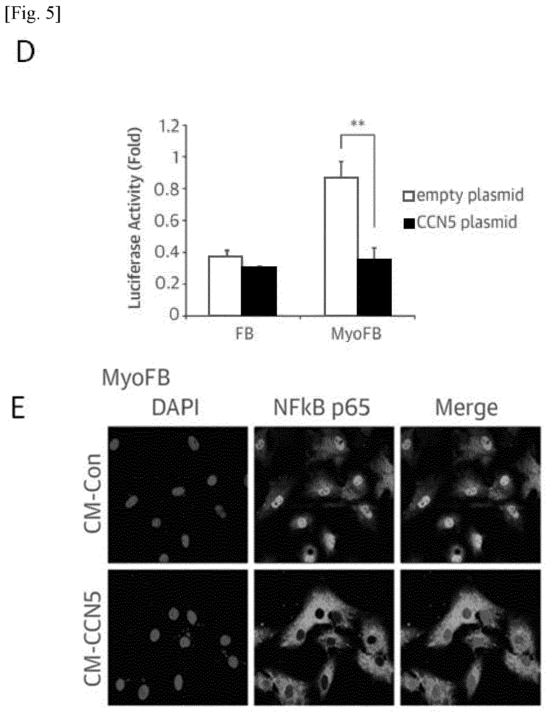

Figs. 5a-5e show the mechanism of selective apoptosis of myofibroblasts by CCN5 protein (n = 3, ^{∗∗} P < 0.01). Fig. 5a is the result of placing fibroblasts and myofibroblasts in a control medium (CM-Con) or a medium including CCN5 protein and culturing the same for 1 day or 2 days, and then separating proteins from the cells and performing western blotting. Fig. 5b is the result of performing the immunochemical method using anti-cytochrome C antibody in the above cell. Fig. 5c is the result of western blotting using NF-κB, vimentin, and anti-α-SMA antibody in the above cells. In addition, Figs. 5d-5e are the results of NF-κB reporter analysis and fluorescence staining with anti-NF-xB antibody in fibroblasts and myofibroblasts.

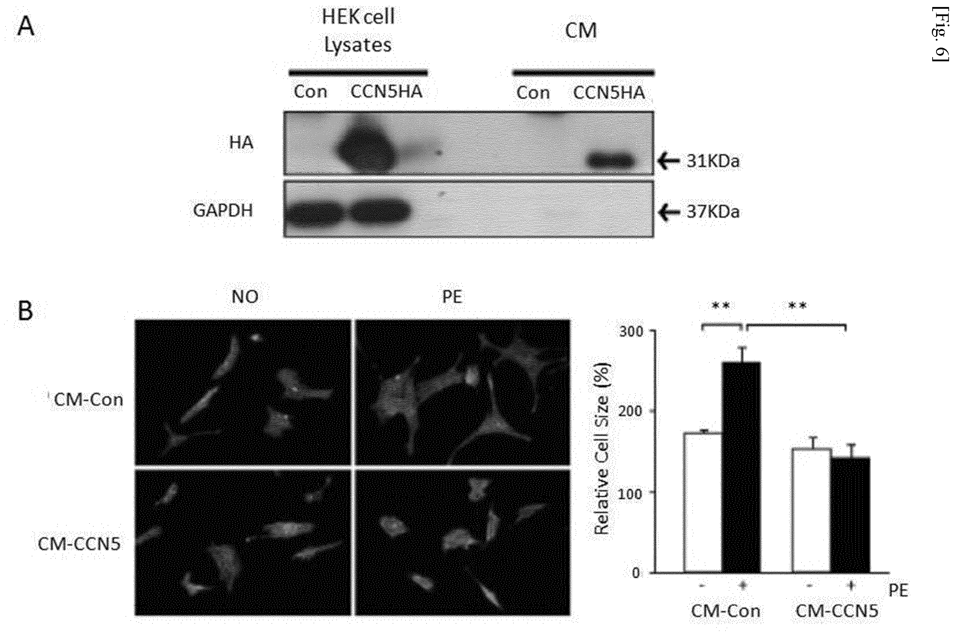

Fig. 6 shows (A) preparation of the cell that overexpresses CCN5 protein and (B) secretion into condition medium liquids and activity thereof.

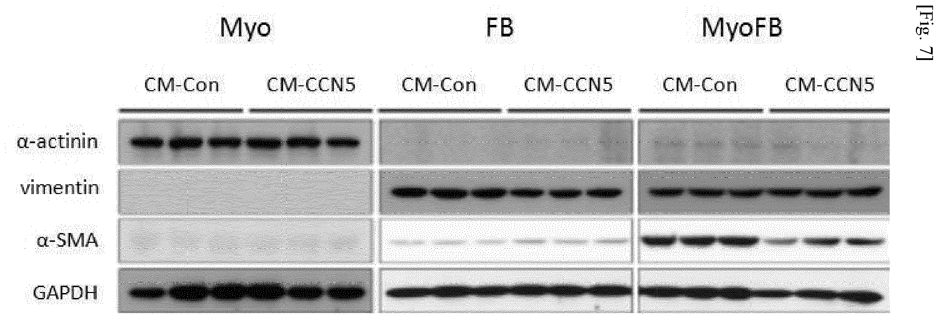

Fig. 7 shows the effects on differentiation induction of myofibroblasts and differentiation of CCN5 protein after treating cardiac muscle cells and myofibroblasts from the cardiac tissue of rats with TGF-β.

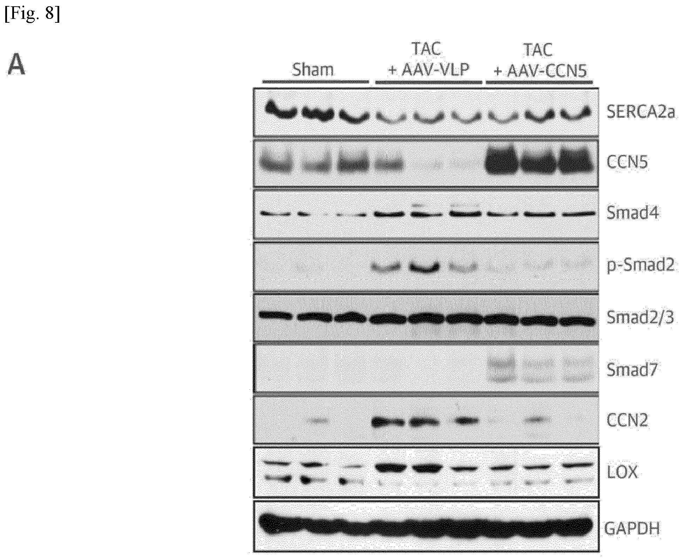

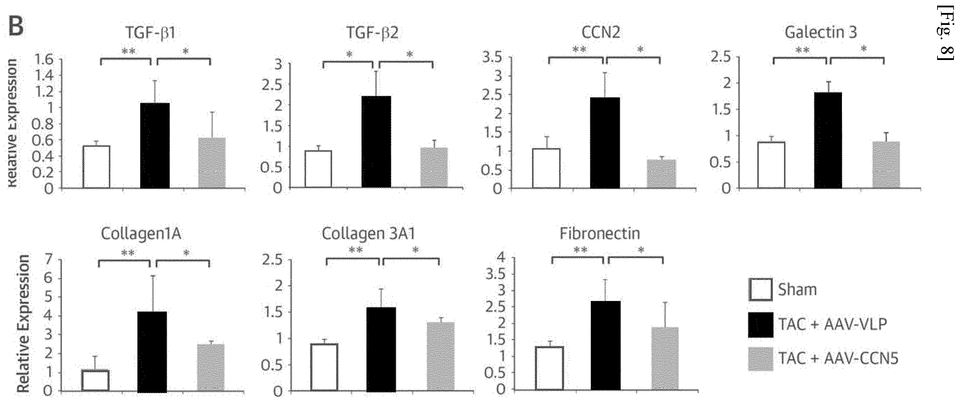

Fig. 8 shows the effects of AAV9-CCN 5 on TGF-β signal transduction in the control group and pressure overload model rats (n = 3, ^{∗∗} P < 0.01).

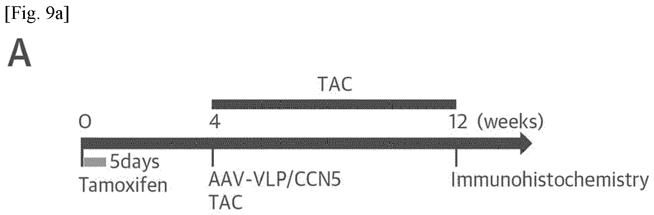

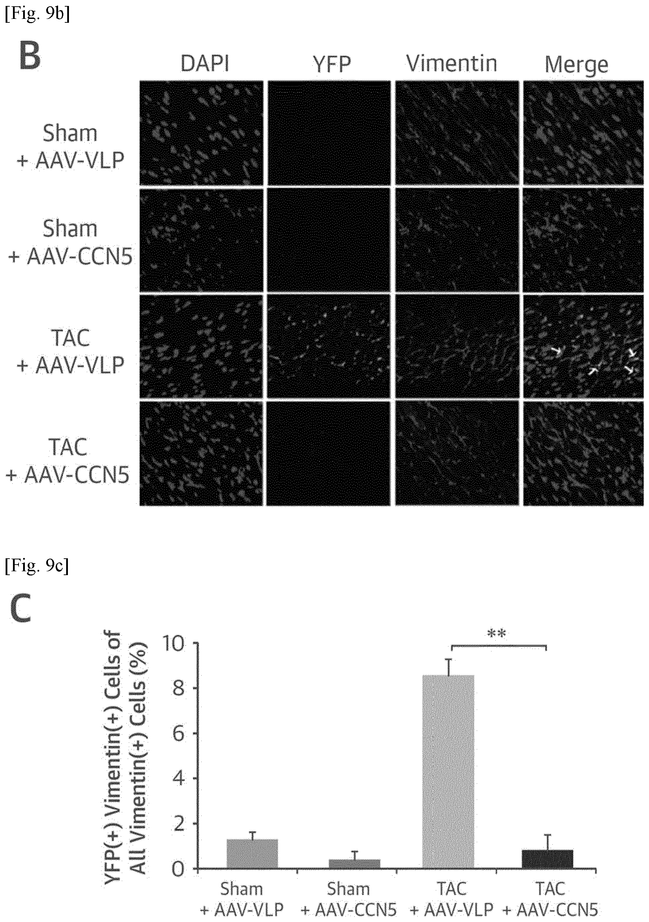

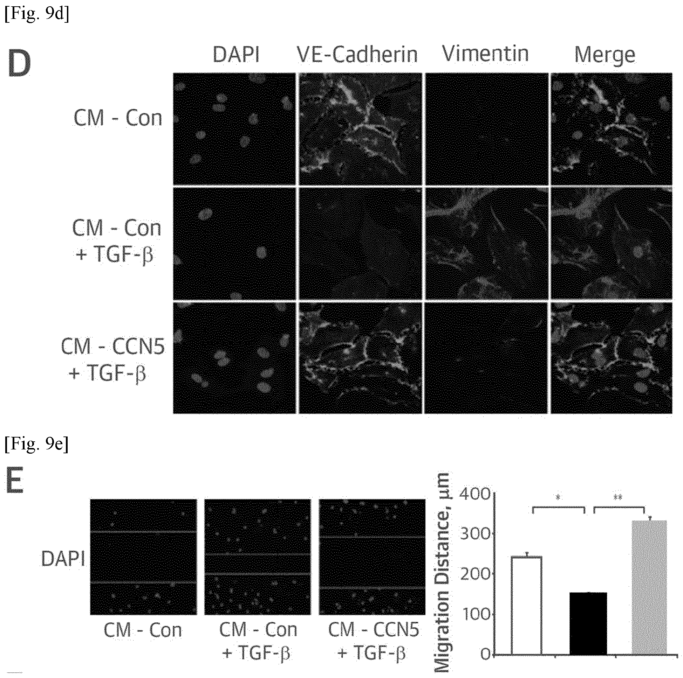

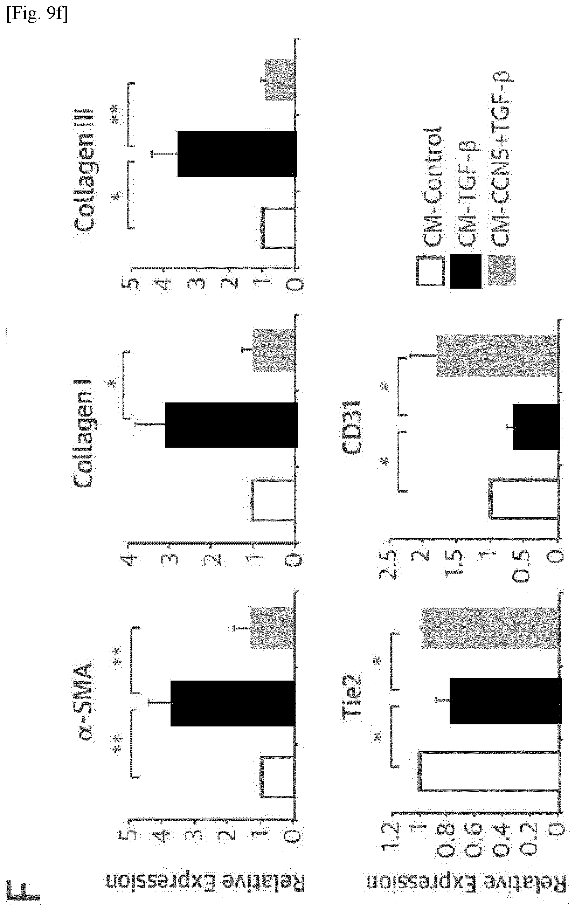

Figs. 9a-9c show the effect of AAV9-CCN5 and CCN5 protein on the result that vascular endothelial cells are transdifferentiated into fibroblasts by the activity of TGF-β (endothelial to mesenchymal transition, EndoMT) and progressed into myofibroblasts *in vivo* and at the cellular level. Fig. 9a shows a schedule of AAV-CCN5 gene injection and gastro-intestinal surgery/aortic cross-stenosis surgery of the Scl-Cre-ERT; R26RstopYFP double transgenic mouse model. Figs. 9b-9c are the result of the immunochemical method by which the cardiac tissue of the mouse model was subjected to sampling 8 weeks after surgery (n = 3, ^{∗}P < 0.05, ^{∗∗} P < 0.01). Fig. 9d is the result of treating TGF-β in human coronary artery endothelial cells (HCAEC) and culturing in a control medium and a medium containing the CCN5 protein for 48 hours, and then carrying out immunochemistry to stain with anti-VE-cadherin and anti-vimentin antibodies. Fig. 9e shows the result of scratching the cultured human coronary artery endothelial cells (HCAEC) by the same area, culturing them under the same medium condition as Fig. 9d, fixing the cells and staining with DAPI, and measuring cell mobility (n = 4, ^{∗} P < 0.05, ^{∗∗} P < 0.01), and Fig. 9f is the result of measurement of mRNA expression levels of α-SMA, collagen I, collagen III, Tie2 and CD31 by qRT-PCR (n = 6, ^{∗} P < 0.05, ^{∗∗} P < 0.01).

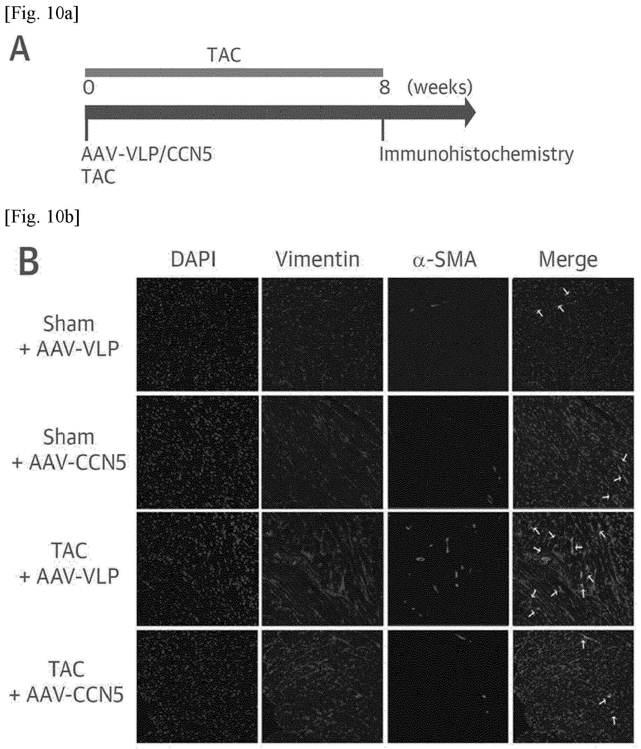

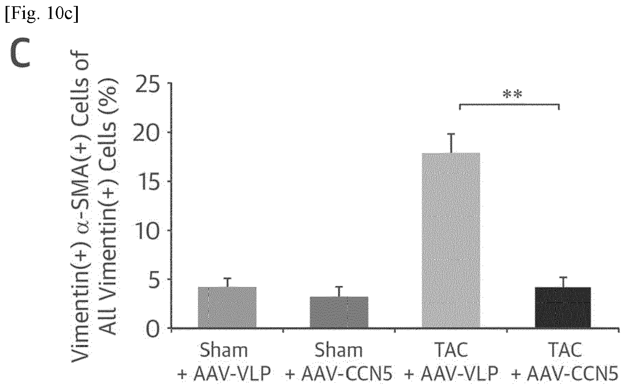

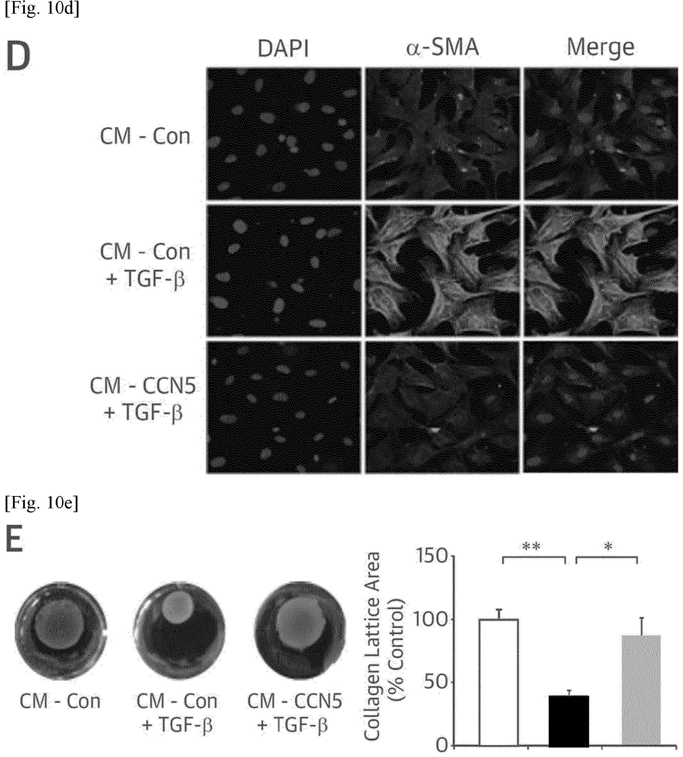

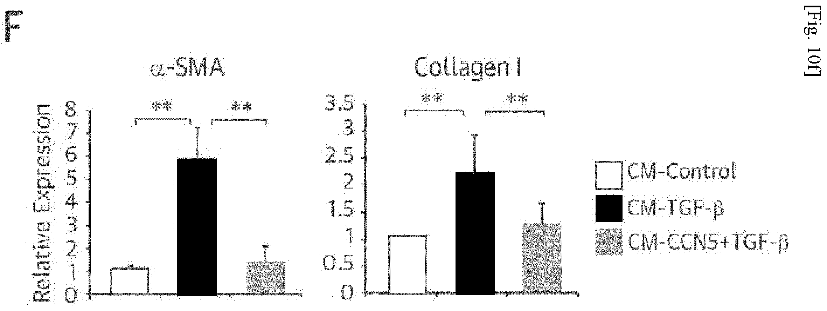

Figs. 10a-10f show the effect of AAV9-CCN5 and CCN5 protein on the transdifferentiation of fibroblasts into myofibroblasts by the activity of TGF-β *in vivo* and at the cellular level. Fig. 10a shows a schedule of AAV-CCN5 gene injection and gastro-intestinal surgery/aortic cross-stenosis surgery of an 8-week old mouse model. Fig. 10b is the result of performing immunochemistry by sampling the cardiac tissue of the mouse model 8 weeks after surgery. Fig. 10c is a graph showing cells expressing α-SMA simultaneously in vimentin-expressing cells (n = 3, P < 0.05, ^{∗∗} P < 0.01). Fig. 10d is the result of treating fibroblasts with TGF-β, culturing in a medium containing the CCN 5 protein for 48 hours, and staining with anti-α-SMA antibody by performing DAPI and immunochemical method. Fig. 10e is the result confirming the inhibition of differentiation of fibroblasts by TGF-β of CCN5 using the collagen gel shrinkage method (n = 3, P < 0.05, ^{∗∗} P < 0.01), and Fig. 10f is the result of measurement of the degree of mRNA expression levels of α-SMA and collagen I by quantitative qRT-PCR (n = 6, P < 0.05, ^{∗∗} P < 0.01).

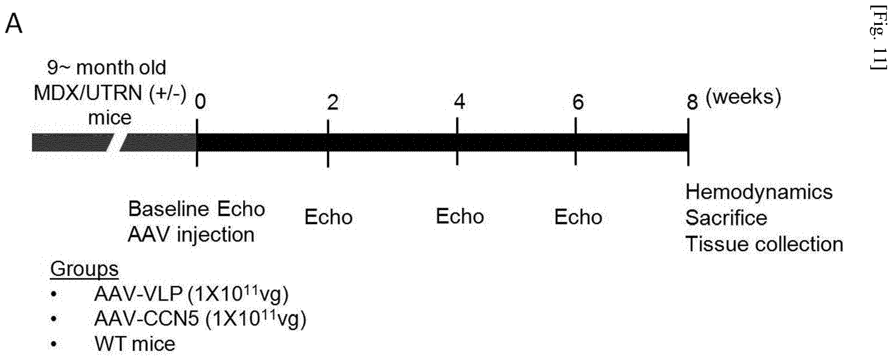

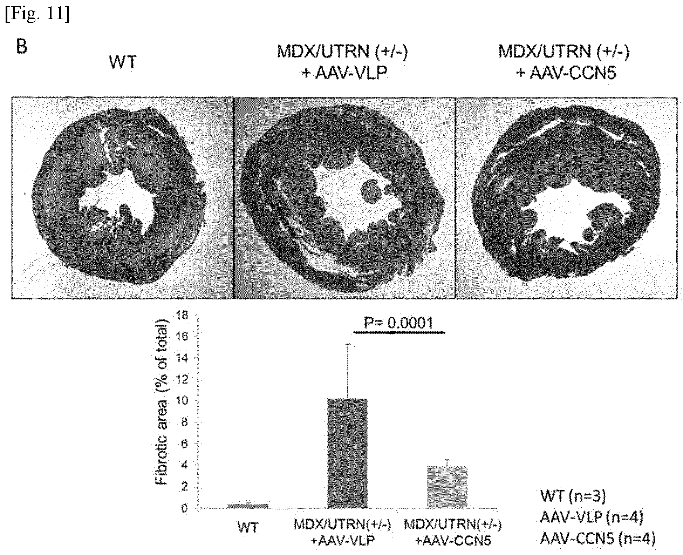

Fig. 11 shows the reversible therapeutic effect and protection of cardiac function against cardiac fibrosis of AAV9-CCN5 in muscle regression dystrophy (Duchenne muscular dystrophy, DMD) model mice. Fig. 11A shows an experimental schedule of inducing aging muscle regressive dystrophic model mice and AAV9-CCN5 gene therapy injection in order to confirm the reversible therapeutic effect. Figs. 11B and Fig. 11C show the degree of fibrosis of the cardiac tissue which was confirmed by using the Masson-Trichrome staining. The range of fibrosis in each experimental group was expressed as a percentage bar graph (n = 3 to 4, p < 0.001).

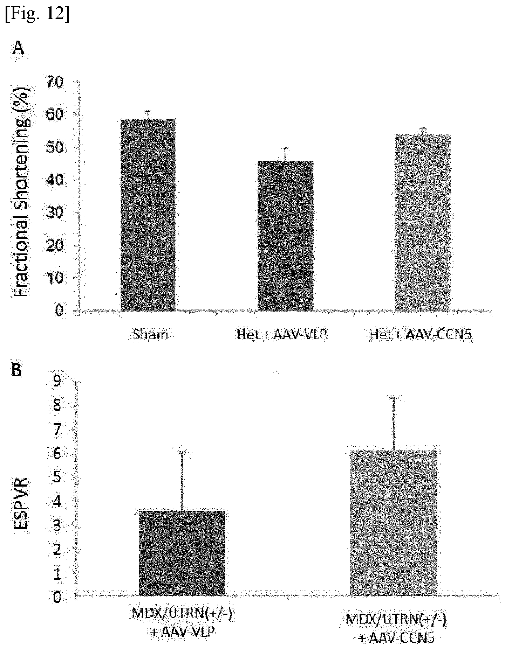

Fig. 12 shows the measurement results for ameliorating cardiac function of AAV9-CCN5 in muscle regression dystrophy (DMD) model mice. Fig. 12A shows the result of measuring the ventricular shortening ratio (n = 3 to 7, p < 0.005). Fig. 12B shows the result of measurement of the hemodynamic function of the heart with the mean end-systolic pressure-volume relationship (ESPVR) (N = 6 to 7, p < 0.05).

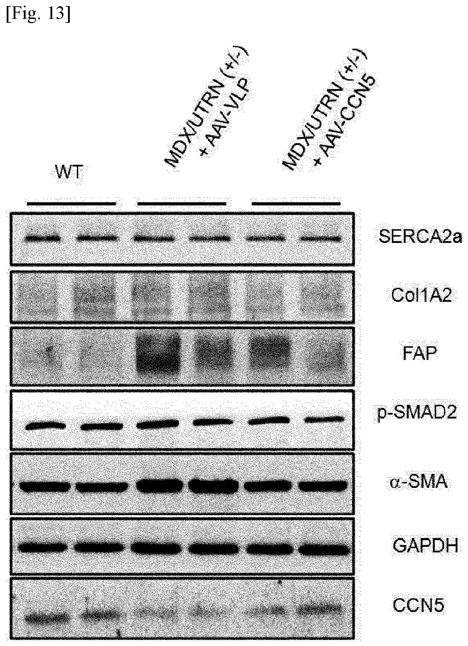

Fig. 13 is the result of confirming changes in the expression of fibrosis-associated proteins by AAV9-CCN5 in muscle regression dystrophy (DMD) model mice by western blotting.

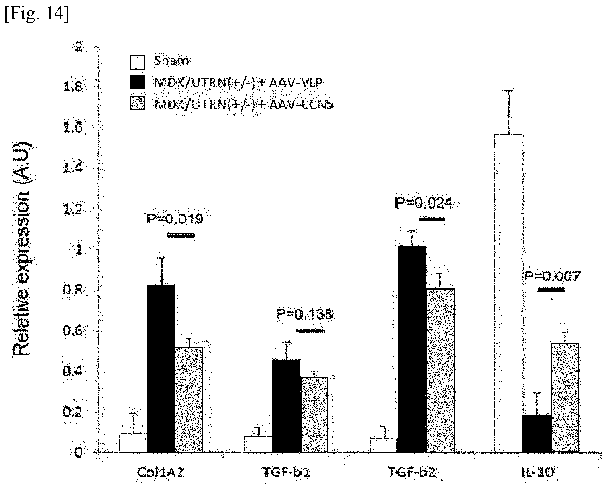

Fig. 14 is the result of confirming changes in the expression of fibrosis-associated genes by AAV9-CCN5 in the muscle regression dystrophy (DMD) model mice by the qRT-PCR method.

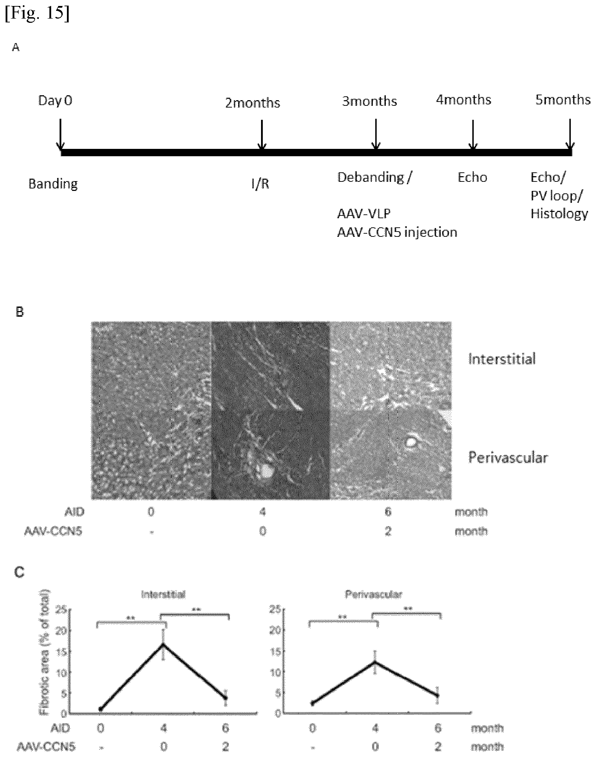

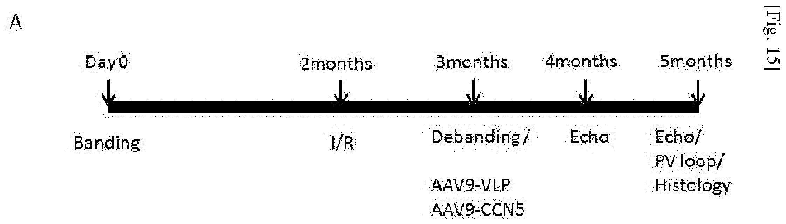

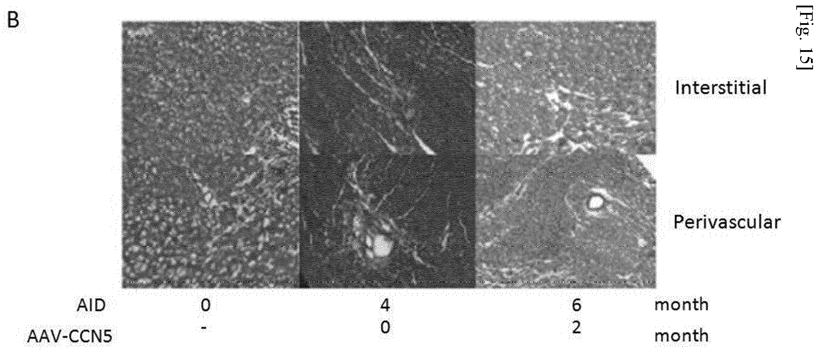

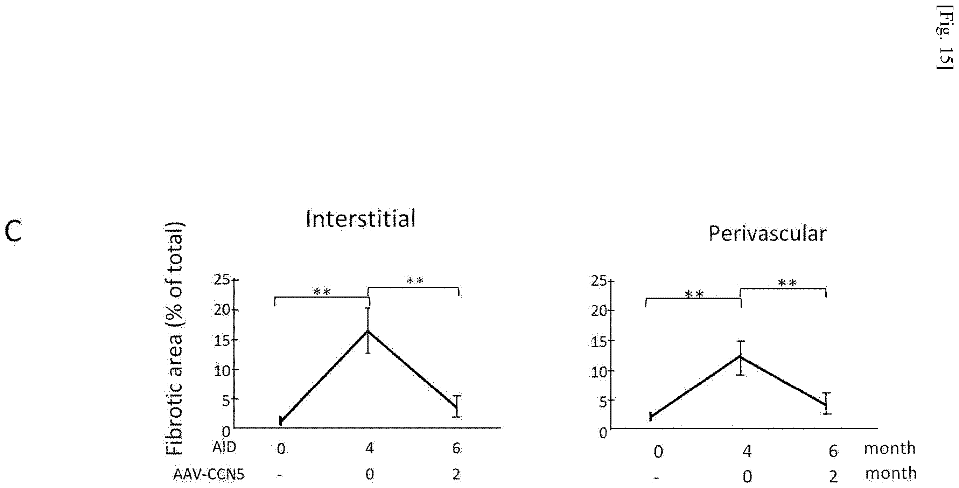

Fig. 15 shows the effect of AAV9-CCN5 on the reversible treatment of pre-existing cardiac fibrosis and cardiac contractility in an Aortic banding-Ischemia-reperfusion-Debanding (AID) heart failure rat model. Fig. 15A shows an experimental schedule of the AID rat model induction and AAV-CCN5 gene therapy injection in order to confirm the reversible therapeutic effect. The upper part of Fig. 15B shows the degree of cardiac fibrosis of the interstitial cells using the Masson-Trichrome staining method. The lower part shows the degree of cardiac fibrosis in the peripheral region of the blood vessel. Fig. 15C shows the relative area where cardiac fibrosis occurred in heart slices (n = 5 to 8, p < 0.05, B and C).

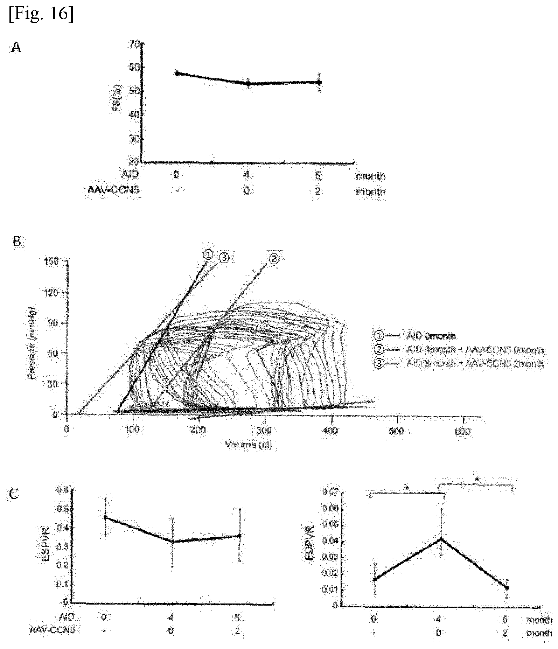

FIG. 16 shows the measurement results for improving the cardiac function of AAV9-CCN5 in the AID rat model. Fig. 16A shows the shortening rate of the ventricle obtained through cardiac ultrasonic analysis (n = 5 to 8, ^{∗∗} P < 0.01). Fig. 16B shows the effect of AAV9-CCN5 on cardiac function in the AID rat model with pressure-volume loop analysis results. Fig. 16C shows the values of end-systolic pressure-volume relations (ESPVR) and end-diastolic pressure-volume relations (EDPVR) which are representative results of the hemodynamic analysis of AAV 9-CCN 5 (N = 6, p < 0.05, B and C). It shows the effect of AAV9-CCN5 on cardiac function in the AID rat model by hemodynamic, pressure-volume loop analysis results. The value of ESPVR indicates the systolic function of the heart, and the value of end-diastolic pressure-volume relations (EDPVR) indicates the diastolic function of the heart.

### Example

All animal experiments were conducted based on the approval and regulation of the Animal Protection and Use Committee of Gwangju Institute of Science and Technology (GIST) and School of Medicine at Mount Sinai. The analysis of the experiments was in accordance with the NIH guidelines on animal protection and use.

### Experimental method

### Construction of animal model (heart failure model) of pressure overload by transverse aortic constriction (TAC)

8- to 10-week old C57BL/6 male mice (body weight 25 to 30 g) from Jackson Laboratory were used for the study. Mice were anesthetized by intraperitoneal injection using a solution made of 95 mg/kg ketamine and 5 mg/kg xylazine. The mice were breathed using an oxygen respirator at a daily breathing rate of 0.2 and a breathing rate of 11 breaths per minute (Harvard Apparatus). In order to observe the aortic arch, the region around the proximal sternum was incised longitudinally 2 to 3 mm. A 27-gauge needle was placed between the innominate artery and the left common carotid artery to connect the aortic arch in the transverse direction. The needle was removed immediately, and the incision site was covered.

### Heart failure model in genetically DMD animals

C57BL/10ScSn (Dmd^{mdx}/Utrn^{tm1Jrs}) was purchased from Jackson Laboratory (Bar Harbor, ME). Male MDX/UTRN (+/-) mice aged 9 months or older were used for experiments to reliably induce muscle regressive dystrophic heart disease. The male mice are a model showing the clinical relevance to Duchenne muscle dystrophy which is a congenital genetic disorder related to the X chromosome of humans.

### Construction of a complex heart failure model (AID model) of pressure overload and ischemia-reperfusion

A complex heart failure model in which pressure overload and ischemia-reperfusion were simultaneously performed in rats was used. Sprague-Dawley rats with a body weight of 180 to 200 grams was used. The second intercostal space was opened with thoracotomy, and the ascending aorta was surrounded by a 4-0 laminated portion like a PE-50 tube with an outer diameter of 0.965 mm, and the PE-50 tube was pulled out. This procedure was used to apply pressure overload to the heart. Two months later, the left anterior descending coronary artery was knotted for 30 minutes and reperfused. In detail, the blood vessel was completely knotted to a 6-0 suture to a point 4 mm below the end of the left heart and released 30 minutes later. Further, one month later, the suture that knotted the ascending aorta was completely released. This complex heart failure model is called Aortic banding-Ischemia-reperfusion-Debanding (AID), which takes 4 months to be constructed.

### Analysis of the function of the heart - Transthoracic echocardiography and in vivo blood dynamics

Model mouse experimental animals were anesthetized via intraperitoneal injection of ketamine at a concentration of 100 ug/g. For two-dimensional images and M-mode tracing, a 15.0 MHz converter (GE Vivid 7 Vision) was used to determine ventricular shortening rates and dimensions to report the shortening of left-ventricular papillary muscle. Rats were anesthetized via isoflurane, after inserting a tube via bronchotomy. The heart was connected to the knot in the left ventricular epicardium using seven sonomicrometer crystals (Sonometrics, London, Ontario, Canada) by performing thoracic incision. The Millar SP-671 pressure converter was inserted through the front wall of the left ventricle. Data were collected using commercially available software SonoLab, and hemodynamic measurements were performed in a range of preload and afterload pressure after temporary closing of the inferior vena cava and aorta. Cardiac function data were analyzed using a series of algorithms generated by MATLAB (v 7.0, The MathWorks, Natick, MA). Echocardiography experiments proceeded throughout the entire experimental period by the same experimenter to increase the accuracy of the experiments. A minimum heart rate of 550 bpm was required to improve the accuracy of the experiments, but this was carried out to include structural and functional evaluation with the bradycardia-related underestimation of the cardiac function minimized. Measurements were performed three times for each mouse, and the average was calculated and expressed as a numerical value. *In vivo* hemodynamic analysis was performed using 1.2 Fr pressure-volume (PV) conductance catheter (Scisense, Ontario, Canada). Mice were anesthetized by intraperitoneal injection with a complex solution of urethane (1 mg/g), etomidate (10 µg/g), and morphine (1 µg/g) and by inserting a tube via bronchial incision, mechanical ventilation was used to set breathing at 125 per minute and the breathing rate at 7 ul/g. PV catheter was positioned inside the left ventricle with a vertex approach, and PV data were analyzed by using IOX2 software (EMKA technologies). The cardiac stress challenge was mesured by injecting dobutamine in a saline solution at an increasing concentration of 1 µg/ml, 10 µg/ml, 100 µg/ml, and 1 mg/ml, and specifically, via a central venous catheter inserted into the right jugular vein with a time interval of 10 minutes in order to normalize hemodynamic parameters.

### Construction and injection of adeno-associated viral vector (AAV)

In order to construct self-complementary AAV (serotype 9), the human CCN5 gene was cloned into the pds-AAV2-EGFP vector. In order to prevent inhibition of virus packaging, eGFP sequence was prepared for AAV vector construction. Recombinant AAV was constructed using 293T cells. The AAV particles in the cell culture solution were collected and precipitated with ammonium sulfate, which were later purified via ultracentrifugation using an iodixanol gradient. The AAV particles were concentrated through a number of dilution replacing iodixanol with Lactate Ringer's solution. The concentration of AAV was quantified using quantitative RT-PCR and SDS-PAGE. AAV-VLP and AAV-CCN5 were injected with 5 × 10¹⁰ - 1 × 10¹¹ virus genomes into the tail vein of model mice and rats.

### Expression of recombinant active CCN5

In order to produce CCN5 protein, the pcDNA3.1-CCN5HA plasmid was used. HEK293 cells were spread on a 60 mm Petri dish at 5 × 10⁵ to stabilize the cells for 1 day. Afterwards, pcDNA3.1-CCN5HA was infected using lipofectin. After 4 hours, in order to remove lipofectin, it was replaced with conditioned media (CM) in which plasma had been removed. Thereafter, the secreted CCN5 obtained after culturing for 24 hours was named as CM-CCN5, which was used for the experiment. Expression of the CCN5 protein was confirmed by western blot and confirmed using an anti-HA antibody tagged with CCN5.

### Separation of resident fibroblast

8-week-old male rats (Damul Science) were subjected to respiratory anesthesia using isoflurane, and their hearts were removed. After inserting the aortic cannula into the aorta, rats were quickly hung to the Langendorff Apparatus and unnecessasry tissues attached to the heart were removed. Using a constant rate perfusion, a physiological fluid (Tyroid buffer; composition: 125 mM/L NaCl, 5 mM/L KCl, 12.5 mM/L HEPES, 11 mM/L glucose, 10 mM/L BDM, 5 taurine, 2.4 MgCl₂). Utilizing the physiological fluid, after sufficiently removing blood, an enzyme solution at 37°C (300 µg/mL collagenolytic enzyme II, 80 µg/mL hyaluronic acid) which was saturated at 100% was perfused for 50 minutes to decompose the cardiac tissue. Through this process, after the cardiac tissue was decomposed, pipetting was carried out several times, and then the tissues and the single cells which were not completely decomposed were separated via the cell filter of 70 µm. The greatest difference between myocardial cells and other cells that make up the heart (fibroblasts, endothelial cells, and smooth muscle cells) is the size of the cells. Utilizing this characteristic, the filtered cell solution was centrifuged at 25 g for 3 minutes to separate myocardial cells. The supernatant was removed and centrifuged for 10 minutes at 250 g to separate fibroblasts, endothelial cells, and smooth muscle cells. The cells thus obtained were mixed in a culture medium containing DMEM (Low glucose /10% FBS/1% antibiotic), spread on a culture dish, and incubated at 37°C in a 5% CO₂ incubator for 4 hours. Fibroblasts have stabilized characteristics of adhering to culture dishes within a short time, which is different from other cells. Therefore, after 4 hours, the medium was replaced, and other cells except fibroblasts, which were cells that were stabilized by adhering to the culture dish, were removed therebetween. After changing the medium the next day, fibroblasts were cultured while replacing the medium every two days (Skrzypiec-Spring, et al. J. Pharmacol. Toxicol. Methods 55: 113-126 (2007)).

### Differentiation induction of fibroblasts into myofibroblasts

Resident fibroblasts isolated from the rat heart were treated with 10 ng/mL TGF-β (Peprotech) and cultured for 2 days at 37°C in a 5% CO 2 incubator. In order to confirm the differentiation into myofibroblasts, α-SMA which is a marker protein was confirmed using immunochemistry (Kovacic J.C., et al. Circulation, Apr 10; 125(14): 1795-1808 (2012)).

### Immunofluorescent staining of transdifferentiation of fibroblasts of CCN5, myofibroblasts of endothelial cells into mesenchymal cells

Fibroblasts and endothelial cells (human coronary artery endothelial cells, HCAECs) were seeded on a 16 mm cover slip and cultured overnight for stabilization, and then treated with 10 ng/mL of TGF-β and CCN5 and cultured for 48 hours. After fixation with 4% paraformaldehyde solution, permeabilization of cell membrane with 0.5% Triton X-100 solution was performed, followed by blocking with 5% BSA solution. Anti-VE-Cadherin (Cell Signaling Technology), anti-vimentin (Santa Cruz), or anti-α-SMA (Sigma) antibody was used for reaction, and Alexa Fluor 488 or Alexa Fluor 594 (Invitrogen) was used as the secondary antibody. The nuclei were stained using DAPI. Cells which were subjected to immunochemistry were analyzed using a fluorescence microscope (Olympus) (Okayama K., et al. Hypertension 59:958-965 (2012)).

### Analysis of collagen gel lattice

In the collagen gel contraction assay, 1 × 10⁶ cells/mL fibroblasts and 1.2 mg/mL collagen solution (Invitrogen) were mixed, and 1 N NaOH (approximately 20 µL) was added to neutralize the pH of the collagen solution. After mixing simply by pipetting, 500 µL of each of the collagen gel suspension was placed in a 24-well plate and polymerized in a 5% CO₂ incubator at 37°C for 30 minutes. After the collagen gel had formed, the control group and treatment groups with TGF-β alone or with CCN5 were cultured in the DMEM medium, respectively. After 48 hours, the extent of the collagen gel constraction was observed and analyzed using the Image J software (Dobaczewski M., et al. Circ. Res. 107: 418-428 (2010)).

### Differentiation induction of vascular endothelial cells into mesenchymal cells (Endothelial-Mesenchymal Transition; EndMT)

EndMT was induced using human coronary artery endothelial cells (HCAECs). HCAECs were purchased through Lonza and cultured in a 5% CO₂ incubator using the EGM-2 (Lonza) culture solution. The HCAEC, which are human-derived cells, were cells with subculture count 3 at the times of purchase, and cells with subculture number of 5 to 7 were used for the experiment. In order to induce EndMT using HCAECs, it is important to maintain the state of cells well through the density of cells during culturing, exchange of the culture solution, *etc.* After spreading HCAECs on the culture dish, it was stabilized for about 12 hours, treated with 10 ng/mL TGF-β, and cultured for 3 days. While EndMT was induced, the culture solution was not replaced, and after 3 days, immunocytochemistry and quantitative-PCR were performed to confirm whether transdifferentiation of HCAECs into mesenchymal cells occurred. Tie 2 and VE-cadherin (CD 31) were used as marker genes of HCAECs, and vimentin, α-SMA, C collagen I, collagen III, and FSP-1 were used as marker genes of mesenchymal cells (Medici D., et al. Biochem. J 437:515-520 (2012)) and (Zeisberg E.M., et al. Nat Med. 13: 952-961 (2007)).

### Cell migration analysis of test tubes

Scratch analysis was performed to measure cell migration ability. HCAECs were placed on a 12-well plate to stabilize the cells overnight. The next day, the culture medium was replaced, and scratches were made on the cells using a tip of 200 µL. The scratched cells were washed with a saline solution, and then cultured with CM-Con or CM-CCN5 treated with 10 ng/mL TGF-β. After 48 hours, the cells were stained with DAPI and analyzed by fluorescence microscopy. The distance traveled by the cells was analyzed using the MetaMorph software (Widyantoro B, et al. Circulation 121:2407-2418(2010)).

### NF-B reporter gene analysis

Rat fibroblasts and myofibroblasts were placed at 3 × 10⁵ cells/well in a 6-well plate and transfected with NF-B reporter plasmid (pBIIx), pRenilla, and other plasmids (empty pcDNA3 vector and pcDNA-hCCN 5) with lipofectamine 2000 (Invitrogen). After 48 hours, the cells were lysed with a passive lysis buffer and centrifuged at 14,000 rpm at 4°C to remove cell debris. Luciferase activity was measured using Dual-luciferase reporter assay system (Promega).

### TUNEL analysis of apoptosis

Cultured myocardial cells, fibroblasts, and myofibroblasts were spread on glass coverslips and stained using DeadEnd Fluorometric TUNEL kit (Promega). It was confirmed that DNA segmentation occurred by apoptosis using terminal deoxynucleotidyl transferase (TdT). In order to confirm TUNEL fluorescence, a Fluoview FV 1000 spherical focus microscope was used (Oberhaus S.M. Methods Mol Biol.218:85-96(2003)).

### Immunohistochemistry

Mouse cardiac tissue was cryopreserved using OCT compound (Tissue-Tek) and then sectioned into 6 µm thick slices. After fixation of the tissue, it was allowed to react using acetone-methanol at -20°C for 20 minutes to become a permeable membrane. Tissues were rehydrated using PBS and blocked with 5% BSA solution for 1 hour. Afterwards, the reaction was carried out using anti-α-SMA, anti-GFP, and anti-vimentin antibodies at 4°C for 12 hours. The tissue reacted with the primary antibody was washed with saline solution and reacted with the secondary antibodies to which fluorescence Alexa546 or Alexa488 was connected for 1 hour at room temperature. For the cardiac tissue examined by fluorescence immunochemistry, the result was confirmed using Fluoview FV 1000 confocal microscope.

### Histopathological staining (Masson-Trichrome staining)

After obtaining the heart muscle from the model animal, it was immediately soaked in an embedding solution at the proper cutting temperature, which was purchased from Fisher Healthcare (Pittsburgh, PA), and then freshly frozen and sectioned into 8 um thick slice. The sectioned samples were observed using an optical microscope after the Masson-Trichrome (Abcam) staining in order to determine the degree of fibrosis.

### Flow cytometry

The separated fibroblast layer was washed with physiological saline containing 0.2% fetal bovine plasma (FBS) and fixed with 2.5% formaldehyde solution. After permeabilizing the cell membrane with 0.5% Triton X-100 solution, the reaction was carried out using anti-α-SMA antibody (Abcam) to which FITC was connected. The cultured fibroblasts and myofibroblasts were washed with 0.2% FBS solution and reacted using an anti-annexin antibody (eBioscience) to which PE was connected. The stained cells were analyzed using Guava easyCye HT (Millipore) (Saada J.I., et al. J. Immunol. 177:5968-79(2006)).

### Western blot analysis

For homogenous solutions of cells and tissues, proteins were separated by size using SDS-PAGE gel and transferred to a PVDF membrane (Millipore). Confirmation of protein expression was performed according to the basic experimental protocol (Kawaki, *et al.,* 2011). The prepared membrane was incubated with anti-CCN2, anti-CCN5 (Lifespan Biosciences), anti-SERCA2a (21st Century Biochemicals), anti-Smad4, anti-p- Smad2, anti-Smad2/3 (Cell signaling), anti-Smad7 (Lifespan Biosciences), anti-LOX (Abcam), anti-α-actinin, anti-α-SMA (Sigma), anti-vimentin, anti-Bcl-2 (Santa Cruz), and anti-Caspase antibodies kit (Cell signaling).

### Quantitative RT-PCR

Real time PCR was performed using QuantiTect SYRB Green real time PCR Kit (Qiagen Ltd.), and the transcription level was analyzed by the same. Trizol (gibco BRL) was used to separate RNA from a cardiac tissue and synthesized it into cDNA. Various quantitative real-time PCR conditions were 37 cycles: 94°C for 10 seconds, 57°C for 15 seconds, and 72°C for 5 seconds. The information on the primers used in the experiment is as shown in Table 1 below.

**[Table 1]**

| **Gene** | **Forward primer** | **Reverse primer** |
|---|---|---|
| Mouse TGF-β1 | 5′-CAACAATTCCTGGCGTTACC TTGG-3′ | 5′-GAAAGCCCTGTATTCCGT CTCCTT-3′ |
| Mouse TGF-β2 | 5′-CGAGCAGCGGATTGAACTGT -3′ | 5′-TTGTGGTGAAGCCACTCC TG-3′ |
| Mouse IL-10 | 5′-TGAGGCGCTGTCGTCATCGA TTTCTCCC-3′ | 5′-ACCTGCTCCACTGCCTTGC T-3 |
| Mouse Galectin 3 | 5′-TTGAAGCTGACCACTTCAAG GTT-3′ | 5'-AGGTTCTTCATCCGATGGT TGT-3' |
| Mouse CCN2 | 5′-CTCCTACTACGAGCTGAACC AG-3′ | 5′-CCAGAAAGCTCAAACTTG ACAGGC-3′ |
| Mouse Collagen 1A | 5′-CCCAAGGAAAAGAAGCACG TC-3′ | 5′-AGGTCAGCTGGATAGCGA CATC-3′ |
| Mouse Collagen 3A | 5′-TGGTAGAAAGGACACAGAG GC-3′ | 5′-TCCAACTTCACCCTTAGC ACC-3′ |
| Mouse Fibronectin | 5′-TTAAGCTCACATGCCAGTGC -3′ | 5′-TCGTCATAGCACGTTGCTT C-3′ |
| Human α-SMA | 5′-GCCCAGCCAAGCACTGTCAG GA-3′ | 5′-TCCCACCATCACCCCCTG ATGTC-3′ |
| Human Collagen I | 5′-CTTGCTTGAAGACCCATGGC -3′ | 5′-TTGGCAGTCTGAGAACCC CA-3′ |
| Human Collagen III | 5′-AAAACGCAAGGCTGTGAGA CT-3′ | 5′-TTTTGTCGGTCACTTGCAC TG-3′ |
| Human | 5′-GCAATGAAGCATGCCACCCT | 5′-GGTAGCGGCCAGCCAGAA |
| Tie2 | GG-3′ | GC-3′ |
| Human CD31 | 5′-GCGAGTCATGGCCCGAAGG C-3′ | 5′-GGTGGTGCTGACATCCGC GA-3′ |
| Rat α-SMA | 5’-TCTGTCTCTAGCACACAACT GTGAATG-3′ | 5′-TTGACAGGCCAGGGCTAG AAGGG-3′ |
| Rat Collagen I | 5′-AATGCACTTTTGGTTTTTGG TCACGT-3′ | 5′-CAGCCCACTTTGCCCCAA CCC-3′ |
| Rat MMP2 | 5'-ACCGTCGCCCATCATCAA-3' | 5′-TTGCACTGCCAACTCTTTG TCT-3′ |
| Rat MMP3 | 5′-TGGACCCTGAGACCTTACCA -3′ | 5′-TGGAAGAGACGGCCAAA ATGA-3′ |
| Rat MMP9 | 5′-TCGAAGGCGACCTCAAGTA GT-3′ | 5′-TTCGGTGTAGCTTTGGATC CA-3′ |
| Rat TIMP2 | 5′-CGTGTCCCAGGGCACAATAA -3′ | 5′-TGTTCAAAGGACCTGACA AGG-3′ |
| Rat TIMP3 | 5′-CGTGTCCCAGGGCACAATAA -3′ | 5′-GATGCAGGCGTAGTGTTT GG-3′ |
| Rat TIMP4 | 5′-CACGTCTGCCACTCTGCTTT- 3′ | 5′-ATCCTTGGCCTTCTCGAAC C-3′ |
| 18S RNA | 5′-TAACGAACGAGACTCTGGC AT-3′ | 5′-CGGACATCTAAGGGCATC ACAG-3′ |

### Statistical processing

Data were expressed as mean ± SD value. Group average was compared using one-way ANOVA (Statview, V5.0, SAS) with Student's t-test or Bonferroni post-test. P <0.05 was considered statistically significant.

### Experimental result

### A. Pressure overload animal model by transverse aortic constriction (TAC)

### Reduction of CCN5 protein expression in cardiac tissue of heart failure

As a result of comparing proteins obtained from the cardiac tissue of heart failure patients provided during heart transplantation surgery and the cardiac tissue of normal people (Table 1) by western blotting, the expression level of the CCN5 protein in heart failure patients was reduced by about 24% (Fig. 1A). The expression of CCN5 in the mouse model of heart failure induced by pressure overload was reduced by about 90% (Fig. 1B). As a result of this experiment, the reduced expression of CCN5 of a cardiac tissue in the state of heart failure was similarly shown in humans and mice, which indicates a clinical correlation between humans and mice. 15 µg of the CCN5 protein extract was used for electrophoresis and confirmed as anti-CCN5 and anti-GAPDH antibodies (^{∗∗} P < 0.01).

### Reversible recovery of cardiac fibrosis by CCN5 gene transfer

The constructed AAV9-CCN5 and the control virus vector AAV9-VLP were injected into the tail vein of the mouse at two concentrations of AAV-CCN5, and the effective concentration was determined by western blotting after 4 weeks. By confirming that injected AAV-CCN5 was expressed specific to the heart, the relationship of pathological activity of cardiac fibrosis and myofibroblasts was studied (n = 6, Error brs = S.D., ^{∗} P < 0.05, Fig. 2). It was confirmed that in the TAC-induced pressure overload heart disease model, cardiac fibrosis occurred 8 weeks after the TAC surgery. AAV-CCN5 and AAV-VLP which is the control group were injected via the tail vein of cardiac fibrosis-induced experimental rats at a concentration of 5 × 10¹⁰ of the viral genome, respectively (n = 16, Fig. 3a). After more than 8 weeks after CCN5 gene transfer, a cardiac tissue was sampled and the degree of tissue fibrosis was quantified via the Masson-Trichrome staining to confirm the therapeutic effect of cardiac fibrosis. In addition, the heart of each experimental group was separated, the fibroblasts were separated using the Langendorff constant-velocity perfusion system, and the degree of cells expressing α-SMA was measured by flow cytometry.

As a result, the extent of fibrosis already progressed in the interstitial tissue and the tissues surrounding blood vessels was similar to that in the group injected with CCN5 in the same degree as in the placebo surgery group (Sham), and it showed a reversible therapeutic effect similar to the degree of being recovered to normal tissue (Figs. 3b-3 c). In addition, the amount of myofibroblasts increased in the fibrotic state of the heart in the AAV9-CCN5 injected group decreased by the amount similar to the placebo surgery group (Fig. 3d). These results demonstrate that CCN5 has a therapeutic effect showing decomposition of fibrotic disorder and reversible recovery of the normal tissue structure from the cardiac tissue with progressed fibrosis. In addition, it was confirmed that the reversible treatment of cardiac fibrosis of CCN5 was related to the decrease of proliferation of fibroblasts by CCN 5, wherein the fibroblasts are pathologic execution cells of occurrence and progression all fibrotic diseases.

The relationship between the treatment of cardiac fibrosis and the protective effect of the cardiac function was examined via echocardiographic examination. Prevention of a decrease in the shortening fraction (FS) of the heart and suppression of an increase in the terminal systole (left ventricular internal dimension in systole, LVIDs) and diastolic left ventricular cavity diameter (LVIDd) were remarkably suppressed in the CCN5 treatment group (AAV-CCN5), compared to the control virus injected group (AAV-VLP). Therefore, the CCN5 protein showed a therapeutic effect for systolic heart failure by preventing the deterioration of cardiac function in the heart failure state through the treatment of pre-existing cardiac fibrosis, and it (n = 16, Error bars = S.D. * P < 0.05, Fig. 3e).

### Selective apoptosis of fibroblasts by CCN5 gene transfer

Correlation analysis was performed to determine whether the recovery of cardiac fibrosis is caused by apoptosis of myofibroblasts. Along with the pressure overload mouse model induction, AAV9-CCN5 and AAV9-VLP were injected, and after 2 weeks, cardiac tissue slices were simultaneously staine with TUNEL staining (red) and anti-α-SMA antibody (green) which is a fibroblast specific protein, and it was confirmed whether the apoptosis of fibroblast cells occurred (Fig. 4b). As a result, there were almost no cells responding to the two staining methods (myofibroblasts underwent apoptosis) in the placebo surgery groups, about 9% in the control group injected with AAV9-VLP to the disease group, and about 72% in the AAV9-CCN5 group (n=3). A remarkably larger number of cells were present in the disease model group injected with AAV9-CCN5 (Figs. 4b and 4c). Therefore, the deterioration of cardiac fibrosis and proliferation of myofibroblasts were proportional, and the reversible therapeutic effect of AAV9-CCN5 injected in an animal model in which cardiac fibrosis has progressed resulted from the apoptosis of myofibroblasts, and it was confirmed that the therapeutic effect of fibrosis by CCN5 gene injection and the decrease of myofibroblasts due to apoptosis were directly related.

In addition, it was confirmed whether CCN5 selectively killed myofibroblasts (Figs. 4d to 4f). Cardiac muscle cells and fibroblasts used in the experiment were obtained from the rat heart, and myofibroblasts were induced by treating fibroblasts with TGF-β. Rat cardiac muscle cells, fibroblasts, and myofibroblasts were cultured in a CM-Con or CM-CCN5 medium, and after 48 hours, staining was carried out using pyknotic nuclei staining with DAPI, TUNEL staining for measuring apoptosis, and staining with an anti-annexin-V (marker for apoptosis) antibody to perform flow cytometry (n = 3, Error brs = S.D., ** P < 0.01). As a result, only in the case of myofibroblasts cultured in the CM-CCN5 medium, it was confirmed that pyknosis increased, and the number of nuclei showing TUNEL fluorescence increased, and the number of annexin-V-positive myofibroblasts increased markedly. These results show the same results as those of animal experiments in which CCN5 selectively causes apoptosis only in myofibroblasts which are the pathological execution cells of cardiac fibrosis.

By taking the two results together, it was found that CCN5 can induce apoptosis of myofibroblasts in the cardiac fibrosis disease model and can show selective mechanisms that do not affect cardiac muscle cells or fibroblasts.

### Investigation of mechanism of selective apoptosis (intrinsic apoptosis pathway) of CCN5 protein

CM-Con or CM-CCN5 was added to myofibroblasts differentiated from fibroblasts under the condition of TGF-β and cultured for 1 day or 2 days, and then proteins were separated from the cells and subjected to western blotting (Fig. 5a). The used antibodies were anti-Bcl2, anti-BAX, anti-Pro-Caspase3, anti-Cleaved Caspase3, Anti-Pro-Caspase7, Anti-Cleaved Caspase7, Anti-Pro-Caspase8, Anti-Cleaved Caspase8, anti-Pro-Caspase9, anti-Cleaved Caspase9, and anti-GAPDH antibodies, which are proteins related to apoptosis. It was confirmed that in the group treated with the CCN5 protein, the expression of BCL-2, a protein that prevents apoptosis, greatly decreased, while that of BAX and caspase 3, 7, and 9, proteins that promote apotosis, increased with time.

In addition, myofibroblasts were treated with CM-Con or CM-CCN5 for 2 days under the same conditions as in the cell culture of western blot, and then immunochemistry was carried out using anti-cytochrome C antibody (Fig. 5b). The arrows in Fig. 5b mean that apoptosis occurred and cytochrome C exited into the cytoplasm from the mitochondria, and the apoptosis of myofibroblasts by treatment of CCN5 protein was confirmed through fluorescent immunochemistry.

Moreover, western blotting using anti-NF-κB, anti-vimentin, and anti-α-SMA antibody in fibroblasts and induced myofibroblasts were carried out under the same conditions as the western blot and immunochemistry described above, and as a result, it was confirmed that expression of NF-κB inside the nucleus which renders resistance to apoptosis was high specifically in myofibroblasts (Fig. 5c). As a result of the NF-κB reporter analysis and immunostaining with an anti-NF-κB antibody, it was confirmed that migration of NF-κB into the nucleus was completely suppressed in the CCN5 protein-treated group (Figs. 5d and 5e). These results prove that the induction of selective apoptosis of myofibroblasts by the CCN5 protein is due to a migration inhibition mechanism of NF-κB in the nucleus (n = 3, Error brs = S.D., ** P < 0.01)

### Confirmation of expression and activity of CM-CCN5

In order to overexpress CCN5 as the protein secreted from cells, HA-tagged Ad-CCN5 plasmid was injected into HEK293 cells and cultured for 24 hours, and then the secreted proteins were obtained from the serum-free cell culture solution (Fig. 6a). The culture medium and the cells were separated, and by using anti-HA antibody, the expression and secreted amount of CCN5 were determined, and it was confirmed that CCN5 secreted into the culture medium by an anti-GAPDH antibody, a cytoplasmic target protein, was not contaminated along with the cytoplasm.

The following experiment was conducted to confirm whether the recombinant CCN5 functionally operated. Cardiac muscle cells of neonatal rats were isolated, cultured in a medium from which plasma had been removed for 12 hours, and then treated with 100 µM of phenylephrine for 24 hours. Phenylephrine induces cardiac cell hypertrophy. It was observed whether cardiac cell hypertrophy is suppressed, by fluorescent immunochemistry using anti-α-actin antibody by simultaneously treating CM-CCN5 at a concentration of 200 ng/mL with phenylephrine in the simultaneous treatment group. The cell surface was measured using the MetaMorph program (n = 50, Error bars = S.D., ** P < 0.01). As a result, the recombinant CCN5 completely inhibited the hypertrophy of the phenylephrine-induced cardiac muscle cells, thus confirming that the recombinant CCN5 functionally operated (Fig. 6b).

### Confirmation of differentiation into myofibroblasts (MyoFB)

Western blot was performed to confirm that differentiated myofibroblasts (MyoFB) were separated and differentiated well in the presence of rat cardiomyocytes (Myo), fibroblasts (FB), and TGF-β, respectively, and it was confirmed using anti-α-actinin, anti-vimentin, anti-α-SMA, and anti-GAPDH antibodies. Even after transdifferentiation of fibroblasts into myofibroblasts, vimentin, which is a marker protein of fibroblasts, did not disappear. Therefore, by confirming that both vimentin and α-SMA were expressed from myofibroblasts, it was confirmed that myofibroblasts were separated and differentiated, thereby improving the reliability of the experiment (Fig. 7).

### Inhibition of signal transduction of fibrosis by CCN5 expressed in the heart

AAV9-VLP, or AAV9-CCN5 (5 × 10¹⁰ virus gene carriers per mouse) were injected into the mouse model 8 weeks after placebo surgery or aortic cross stenotic surgery (TAC), and after additional 8 weeks, the expression of a protein related to signal transduction of fibrosis was confirmed using western blot in the heart of a sacrificed model (n = 3, Figs. 8a and 8b). The protein obtained from the heart tissue was used at a concentration of 50 µg and was confirmed using anti-SERCA2a, anti-CCN5, anti-Smad4, anti-p-Smad2, anti-Smad7, anti-CCN2, anti-LOX, and anti-GAPDH antibodies. As a result, in the control group, Smad2 involved in the activation of TGF-β signaling was phosphorylated, but the expression of p-Smad2 was inhibited in the CCN5-injected group. In addition, Smad7, which is involved in inhibiting TGF-β signaling, was expressed only in the CCN5 group. Lysyl oxidase (LOX), an important drug target for inhibiting the progression of fibrosis, is an enzyme that crosslinks and polymerizes collagen secreted in the process of fibrosis and plays an important role in curing the external environment of tissues. Although it increased in the control group, it was confirmed that it decreased considerably in the CCN5-injected group. Moreover, it was possible to predict the cardiac function-improving effect of systole by the contractile force of the heart due to the result of an increase of the SERCA2a protein responsible for the pumping function of blood circulation by increasing the systolic force of the heart.

RNA was extracted using the same cardiac tissue, cDNA was synthesized, and the expression level of mRNA involved in the signal transduction of fibrosis was determined by quantitative RT-PCR (Fig. 8b). mRNAs of TGF-β1, TGF-β2, CCN2, Galectin 3, collagen 1A, collagen 3A1, and fibronectin were measured. As a result of analyzing the target gene of cardiac fibrosis using quantitative RT-PCR, it was confirmed that the gene which directs the signal transduction of fibrosis such as TGF-β type, CCN2, Galectin 3, *etc.* and the fibrotic protein genes which increased through the transcriptional mechanism thereof increased in the control group, they decreased in the CCN5-injected group (n = 3, ** P < 0.01).

### Inhibition of transdifferentiation of endothelial cell into myofibroblasts by CCN5

As a result of recent studies, it was found that fibroblasts which are proliferated in the course of cardiac fibrosis are formed in part by transdifferentiation from endothelial cells (EndoMT) and promote the formation of myofibroblasts, and it was confirmed whether CCN5 inhibits such transdifferentiation using the Scl-Cre-ERT; R26RstopYFP double transgenic mouse model.

In the Scl-Cre-ERT; R26 Rstop YFP double transgenic mouse model, tamoxifen was treated for 5 days, and after 4 weeks, AAV9-VLP, or AAV9-CCN5 (5 × 10¹⁰ virus gene carriers per mouse) were injected, and simultaneously, placebo surgery or transverse aortic constriction (TAC) surgery were performed (Fig. 9 a). After 8 weeks of TAC surgery, cardiac tissues were sampled, and immunochemistry was performed (Figs. 9b and 9c). The cross-section of the cardiac tissue was stained with anti-YFP (green) and anti-vimentin (red) antibodies, and cells simultaneously expressing tamoxifen-induced YFP and vimentin which is a target gene of fibroblasts were measured. Cells expressing YFP and vimentin at the same time can be cells in which endothelial cells are converted to mesodermal cells. As a result, it was confirmed that about 8.5% of the cells expressed YFP and vimentin at the same time in the AAV-VLP-injected group, but only about 1% of the cells expressed simultaneously the YFP and vimentin in the AAV-CCN5-injected group. The above results demonstrate that CCN5 is capable of inhibiting transdifferentiation and proliferation of myofibroblasts, which are the causative cell of fibrosis, from various precursor cells.

In addition, depending on the site of damage of tissues and organs, along with fibroblasts and pericytes, endothelial cells are transdifferentiated into fibroblasts to finally differentiate into myofibroblasts due to fibrotic inducing factors such as TGF-β. It was confirmed whether CCN5 inhibited endothelial cells from being transdifferentiated into mesodermal cells by TGF-β (Fig. 9d). In order to induce endothelial cells into mesodermal cells through transdifferentiation, human coronary artery endothelial cells (HCAECs) were treated with 10 ng/mL TGF-β2 for 72 hours. The cells were cultured in a medium containing a control medium (CM-Con) and 200 ng/mL CCN5 (CM-CCN5) to observe the results. Immunochemistry was performed to stain with anti-VE-cadherin and anti-vimentin antibodies, and the nuclei were stained with DAPI. As a result, in the case of treating human coronary artery endothelial cells (HCAECs) with TGF-β, α-smooth muscle actin (α-SMA) and vimentin, which are markers for myofibroblasts, were both expressed, and in the group simultaneously treated with CCN5, expression was suppressed.

Moreover, when endothelial cells undergo transdifferentiation, the characteristics similar to myofibroblasts develop and have the ability to migrate to the wound site, and it was confirmed whether CCN5 suppresses this by using flow cytometry (Fig. 9e). After spreading and stabilizing HCAECs on a culture dish, scratches were made using a 200 µL pipette tip, and then the cells were cultured under the conditions of immunochemistry shown in Fig. 9d. After 48 hours, the cells were fixed and stained with DAPI, and the extent to which the cells migrated was measured. As a result, in the group treated with TGF-β, the migratory capacity of the cells increased compared with the control group, and in the group simultaneously treated with TGF-β and CCN5, cell migration did not occur. In addition, after extracting RNA by culturing the endothelial cells under the same conditions as in the immunochemistry of Fig. 9d, cDNA was synthesized, and quantitative RT-PCR was performed to measure the degree of mRNA expression of α-SMA, collagen I, collagen III, Tie2 and CD31 to analyze the gene expression (Fig. 9f). As a result, the expression of α-SMA, collagen I, and collagen III, which are genes related to fibroblasts induced by TGF-β in the group simultaneously treated with CCN5, and the expression of Tie 2 and CD31 genes, which are inherent of endothelial cells, were shown to be similar to the TGF-β untreated control group (n = 6, Error bars = S.D., * P < 0.05, ** P < 0.01). Therefore, it was shown that CCN5 inhibited the transdifferentiation mechanism from endothelial cells into mesodermal cells to suppress fibrosis.

### Inhibition of differentiation of fibroblast into myofibroblast by CCN5

The following experiment was conducted to confirm whether CCN5 inhibits differentiation of fibroblasts into myofibroblasts. 8-week-old mouse model was injected with AAV9-VLP, or AAV9-CCN5 (5 × 10¹⁰ virus gene carriers per mouse), at the same time performing placebo surgery or transverse aortic constriction (TAC) surgery, and after 8 weeks, immunochemistry was performed using the cardiac tissue (Fig. 10a). The cross section of the cardiac tissue was stained with anti-vimentin (red) and anti-α-SMA (green) antibodies. Cells that simultaneously express vimentin and α-SMA can be cells in which conversion to fibroblasts occurred (Fig. 10 b). Cells expressing α-SMA simultaneously among vimentin-expressing cells were analyzed and shown in a graph (n = 3, Error bars = S.D., ** P < 0.01). As a result, α-SMA and vimentin were simultaneously expressed in about 17% of the cells in the AAV9-VLP-injected group, whereas in the AAV9-CCN5-injected group, about 4% of the cells expressed α-SMA and vimentin at the same time. This is the result showing that the increased expression of CCN5 in the heart effectively suppresses the pathological mechanism by which fibroblasts causing fibrosis are differentiated and proliferated into myofibroblasts (n = 3, Error bars = S.D., ** P < 0.01, Fig. 10c).

The following experiment was conducted to confirm that recombinant CCN5 protein also suppresses the differentiation of myofibroblasts by TGF-β. In order to induce fibroblasts to differentiate into myofibroblasts, 10 ng/mL TGF-β was treated for 48 hours. The cells were cultured in a control group medium and a 200 ng/mL CCN5 medium, respectively, and the results were observed. There were stained with an anti-α-SMA antibody, and the nucleus was stained with DAPI to perform immunochemistry. It was confirmed that the increase in the protein expression level of α-smooth muscle actin, which provides contractile force of myofibroblasts in the group simultaneously treated with CCN5, was suppressed (Fig. 10d).

Since TGF-β increases the contractile force of the collagen gel, using the collagen gel contraction assay, it was confirmed that CCN5 inhibits differentiation of myofibroblasts by TGF-β (Fig. 10d). Collagen lattice gel was made using fibroblasts and collagen, and cultured under the same conditions as in the experiment related to Fig. 10d, and then the size of the collagen gel was measured 48 hours afterwards. As a result, it was confirmed that the contractile force of the collagen gel due to TGF-β significantly decreased in the group treated with CCN5.

Quantitative RT-PCR was performed to measure mRNA expression levels of α-SMA and collagen I (Fig. 10f). Fibroblasts were also cultured under the same condition as in the experiment related to Fig. 10 to extract RNA, cDNA was synthesized, and quantitative RT-PCR was performed to measure the degree of mRNA expression of α-SMA and collagen I. As a result, it was found that the expression of α-smooth muscle actin and collagen 1 genes, which is a specific transcriptional mechanism of TGF-β-induced myofibroblasts, was suppressed to the same level as fibroblasts in the group simultaneously treated with CCN5 (n = 6, Error bars = S.D., * P < 0.05, ** P < 0.01). Therefore, it was demonstrated that CCN5 effectively inhibits differentiation into and production of myofibroblasts by TGF- β.

### B. Genetic Duchenne muscular dystrophy heart failure model (DMD model)

### The therapeutic effect of AAV-CCN5 on cardiac fibrosis in DMD model

The therapeutic effect of AAV-CCN5 in aged MDX/UTRN (+/-) mice, which are Duchenne muscular dystrophy model animals, was tested according to the schedule in Fig. 11a. The extent of fibrosis was confirmed via the cryo-cutting method of the cardiac muscle using the Masson-Trichrome staining (Fig. 11b). As a result of measuring the range of cardiac fibrosis, the range of fibrosis decreased by 2.6-fold on average in the AAV-CCN5-injected group (3.39% +/- 0.58) compared to the AAV-VLP-injected group (10.14% +/- 5.11) (Fig. 11b). Suppression of interstitial fibrosis accumulation in the heart injected with AANV-CCN5 showed improved effect of structural remodeling and a therapeutic effect of normalizing the disposition of the heart muscle tissue (n = 3 to 4, p < 0.001).

### Improvement treatment of cardiac function by AA V-CCN5 in DMD model

Echocardiography was performed 8 weeks after injection of AAV-VLP and AAV-CCN5. As a result of measuring the ventricular shortening ratio, a therapeutic effect of ventricular shortening by CCN5 were shown as 58.70% ± 1.05 (n = 3), 45.75% ± 1.29 (n = 7) and 53.88 ± 1.21 (n = 6) in the normal sham-operated WT (+/-) group, the AAV-VLP group, and the AAV-CCN5 group, respectively (P < 0.005, Fig. 12a). In addition, as a result of measuring the hemodynamic function of the heart, the average end-systolic pressure-volume relationship (ESPVR) of the AAV-CCN5-injected group, was 3.54 ± 2.49 (n = 7) whereas that of the AAV-CCN5-injected group was 6.10 ± 2.25 (n = 6). This result demonstrates the therapeutic effect of overexpressed CCN5 protein which improves the terminal systolic elasticity and contractility of the heart (p < 0.05, Fig. 12b).

### Inhibiting effect of AA V-CCN5 on expression of genes related to cardiac fibrosis

In the DMD model animals, for the normal mice and aged MDX/UTRN (+/-) mice injected with AAV-VLP and AAV-CCN5, SERCA2a which is related to contraction of cardiac muscle; Col1A2, fibroblast activating protein (FAP), p-SMAD and α-SMA which are genes related to fibrosis; and CCN5 protein were western blotted. In the AAV-CCN5-injected group, the expression of SERCA2a protein which is important for cardiac systole increased, and the expressions of Col1A2, fibroblast activating protein (FAP), p-SMAD, and α-SMA, which are fibrosis marker proteins, decreased (Fig. 13). As a result of determining the expressions at the genetic level using qRT-PCR, it was confirmed that the expression of Col1A2 and TGF-β1 decreased in the AAV-CCN5-injected group, and the expression of IL-10 which is an anti-inflammatory cytokine increased (Fig. 14). Therefore, in the AAV-CCN5-injected group, it was found that the overexpressed CCN5 protein was effective in cardiac fibrosis treatment through protein and gene expression experiments.

### C. Aortic banding-Ischemia-reperfusion-Debanding (AID) heart failure model

### Therapeutic effect on cardiac fibrosis by CCN5 gene transfer

The diastolic heart failure model used herein is a model constructed on rats through the Aortic banding-Ischemia-reperfusion-Debanding (AID) surgical method. This model better reflects the situation of heart failure patients than the pressure overload model commonly used for heart failure studies. That is, many heart failure patients often experience pressure overload due to high blood pressure, *etc.* and ischemia due to angina pectoris, myocardial infarction, *etc.* at the same time. However, although the pressure load is removed by surgical method and drug treatment, and perfusion of the coronary arteries is achieved, the condition of the heart is not yet improved but progresses to heart failure (Fig. 15a). The present inventors discovered that cardiac fibrosis had progressed seriously from the heart of the AID model, thereby greatly reducing the diastolic function of the heart. On the other hand, the decrease in systolic function of the heart was not large enough to have statistical significance. Therefore, the AID model represents the situation of heart failure patients better and especially has the characteristics of heart failure with preserved ejection fraction among others. After dividing the AID heart failure rat model into a control group and a treatment group, AAV 9-CCN 5(1 × 10¹¹ virus genomes per mouse) were injected into the tail vein and analyzed 2 months later. Cardiac sections were stained with Masson-Trichrome and observed under a microscope. Collagen that was secreted in cardiac fibrosis was blue-stained by the Masson-Trichrome staining.

As a result, it was confirmed that significant cardiac fibrosis progressed in interstitial and perivascular regions in AID rats. However, in rats injected with AAV-CCN5, cardiac fibrosis was treated to the extent similar to the rats without surgery (n = 5 to 8, p < 0.05, Figs. 15b and 15c).

### Confirmation of systolic and diastolic function of the heart using hemodynamic analysis

When CCN5 was overexpressed in cardiac muscle cells using AAV-CCN5, fractional shortening obtained by echocardiographic analysis in the AID model showed no significant difference between the experimental groups but showed a function close to normal (Fig. 16a). The CCN5 effect was confirmed in the AID heart failure model through hemodynamic analysis. In this analysis, the value of End-Diastolic Pressure-Volume Relations (ESPVR) is proportional to the contractile function of the heart (contractility), and the value of (EDPVR) is inversely proportional to the diastolic function of the heart (compliance). The experimental results showed an insignificant decrease ESPVR and a significant increase in EDPVR in the AAV-CCN5-injected group in AID rats (n = 6, p < 0.05, Figs. 16b and 16c). This means that, compared to normal rats, AID rats do not have a significantly decreased contractile force, but significant reduced diastolic function. No reduction in this diastolic function was observed in the rats injected with AAV-CCN5. These results mean that the reduced diastolic heart function can be restored in AID model rats overexpressing the CCN5 protein.

### Analysis of results and further discussion

Fibrotic diseases, which are irreversible disease have a problem in that they are diagnosed after the occurrence of diseases. Any successful remedial therapeutic agent has not been developed up to date. As a characteristic of fibrotic diseases, fibroblasts, which are pathogenic execution cells, play a central role in the occurrence and progression of the diseases regardless of the type of tissues and organs. Myofibroblasts have characteristics of cells which are temporarily induced and disappear in the healing process of normal wounds, but in the fibrotic disease state, they have a continuous proliferation function and activity by acquiring a mechanism to prevent apoptosis. Such persistent activity of fibroblasts is shown in the path of disease common in diseases in which fibrosis progresses. Pathogenic myofibroblasts have a function of secretory cells that overproduce and accumulate the fibrotic extracellular matrix, a function of inflammatory cells of the influx of inflammatory immune cells and auto-secretion of inflammatory substances, and a cellular function of signal transduction which disrupts the function of structural compositional cells due to the abnormal connection to the surrounding cells and surrounding secreted substances. Consequently, sustained proliferation and activation of myofibroblasts are not only the causes of fibrosis causing tissue remodeling but also play a role in promoting the process of reactive fibrosis by the function of inflammatory cells.

Through the present studies, it was first revealed at the cellular level and at the biological level of the disease state that the CCN5 protein causes selective killing of myofibroblasts, which are the central cells of the cardiac fibrosis process. The CCN5 protein can control the continuous pathological activity of myofibroblasts and promote selective apoptosis to provide a method for reversibly treating pre-existing cardiac fibrosis with substances in the human body. In particular, the development of a drug that regulates the apoptosis of fibroblasts for therapeutic purposes, has not been successful, but selective apoptosis of fibroblasts by the CCN5 protein has a characteristic of the human body imitation mechanism occurring in the resolution period of the normal wound-healing process. These facts suggest that the mechanism by which only pathogenic myofibroblasts are killed without affecting fibroblasts which are precursor cells and cardiac muscle cells can minimize side effects and is a new biomimetic therapy. Therefore, for the treatment of pre-existing fibrosis, the dissolution of the fibrotic matrix and normal structural composition are restored by the CCN5 protein activity, and thereby a treatment method for fibrotic disease which is an incurable disease including cardiac fibrosis can be provided.

CCN5 is a matricellular protein that is secreted extracellularly and shows therapeutic activity, and therefore, CCN5 has the potential to be able to treat various disease conditions and a wide range of sites by a drug delivery method such as protein formulation, a gene therapeutic agent, a cell therapeutic agent in which a gene is amplified, *etc.* On the basis of these characteristics, as a result of expressing CCN5 in the heart via AAV9-CCN5 gene transfer in various animal models with a considerable progress of cardiac fibrosis, the pre-existing fibrous tissue is reversibly treated. The reversible treatment mechanism of CCN5 reproduced the *in vivo* treatment effect through selectively inducing apoptosis of myofibroblasts like in the experiments at the cellular level. In particular, the therapeutic effect for cardiac fibrosis was confirmed through treating interstitial fibrosis and perivascular fibrosis of the cardiac muscle tissue. In addition, as a result of the therapeutic effect of CCN5 through protein and gene analysis, each of the decrease in the LOX enzyme which increases rigidity by causing crosslinking of collagen (Rosin N.L., et al., Am. J Pathol. 185(3):631-642(2015), the increase in the expression of the Smad7 protein which is an inhibitor of TGF-β signaling (Wei L.H., Huang X.R., et al. Cardiovasc. Res. 1;99(4): 665-73(2013)), the decrease in Galectin-3 that promotes inflow of inflammatory immune cells and proliferation of myofibroblasts (Ho J.E., Liu C., et al. J Am. Coll. Cardiol. 60(14):1249-1256(2012)), and the decrease in the expressions of TGF-β1 and 2 was the drug target of fibrosis treatment, which showed that the treatment mechanism of CCN5 is strong. Therefore, the CCN5 protein can provide an effective treatment means for the treatment of various cardiac fibrotic diseases.

Cardiac dysfunction accompanied by cardiac fibrosis is the biggest disease cause of heart failure with preserve ejection fraction (HFpEF). Rigidification of the ventricle causes problems in cardiac muscle cell atrophy and cardiovascular contraction and relaxation due to abnormalities in the relaxing function of the heart muscle and an increase in the extracellular interstitial tissue. As a result, induction of hypoxia, impairment of cellular energy metabolism, and continued influx of inflammatory cells progress necrosis of cardiac muscle cells, and heart failure with preserve ejection fraction (HFpEF) progresses into heart failure with reduced ejection fraction (HFrEF). In addition, in the case of heart failure with reduced ejection fraction (HFrEF) in which a large number of deaths of cardiac muscle cells occurs similar to myocardial infarction, replacement fibrosis occurs at the damaged site, but interstitial fibrosis progresses at the boundary and distant tissues. Consequently, cardiac fibrosis can act as a risk factor to exacerbate systolic heart failure conditions. In the present studies, the CCN5 protein is found to be effective in the reversible treatment of cardiac fibrosis and the recovery and protective effect in systolic heart failure in the pressure overload (TAC) model and muscle Duchenne muscle dystrophy model (DMD) which is a rare disease through *in vivo* models. In addition, the CCN5 protein was able to reversibly treat cardiac fibrosis and restore the reduction of diastolic cardiac function through a rat AID model experiment in which diastolic heart failure occurs. This result indicates that the CCN5 protein can be developed as a therapeutic agent for reversible treatment of cardiac fibrosis or systolic heart failure (HFrEF) and diastolic heart failure (HFpEF) accompanied with cardiac fibrosis.

### References

Bharath Ambale-Venkatesh and Joao A.C. Lima, Cardiac MRI: a central prognostic tool in myocardial fibrosis. Nat. Rev. Cardiol. 2015:12:18-29.
Borlaug BA, The pathophysiology of heart failure with preserved ejection fraction. Nat Rev Cardiol. 2014;11(9):507-515.
Burchfield JS, Xie M, Hill JA, et al. Pathological ventricular remodeling: mechanisms: part 1 of 2. Circulation. 2013;128(4):388-400.
Butler J, Fonarow GC, et al.Developing therapies for heart failure with preserved ejection fraction: current state and future directions. JACC Heart Fail. 2014;2(2):97-112.
Chung ES, Miller L, et al. Changes in ventricular remodelling and clinical status during the year following a single administration of stromal cell-derived factor-1 non-viral gene therapy in chronic ischaemic heart failure patients: the STOP-HF randomized Phase II trial. Eur Heart J. 2015;36(33):2228-2238.
Coen, M., Gabbiani, G. & Bochaton-Piallat, M. L. Myofibroblast-mediated adventitial remodeling: an underestimated player in arterial pathology. Arterioscler. Thromb. Vasc. Biol 2011;31: 2391-2396.
Collier P1, Ledwidge M, McDonald K, Diagnostics and therapeutic interventions in myocardial interstitial disease, a previously neglected pathology. QJM. 2012; 105(8):721-724.
Darby IA, Laverdet B, et al. Fibroblasts and myofibroblasts in wound healing. Clin Cosmet Investig Dermatol. 2014;7:301-311.
de Jong S, van Veen TA et al., Fibrosis and cardiac arrhythmias. J Cardiovasc Pharmacol. 2011;57(6):630-638.
Dobaczewski M, Bujak M, Li N, et al. Smad3 signaling critically regulates fibroblast phenotype and function in healing myocardial infarction. Circ Res 2010;107:418-428.
Drakos SG, Kfoury AG,et al. Impact of mechanical unloading on microvasculature and associated central remodeling features of the failing human heart. J Am Coll Cardiol. 2010 27;56(5):382-391.
Dufferield JS, Lupher M, et al. Host responses in tissue repair and fibrosis. Annu. Rev. Pathol. Mech. Dis.2013;8:241-276.
F.G. Spinale, Myocardial matrix remodeling and the matrix metalloproteinases: influence on cardiac form and function. Physiol Rev. 2007;87: 1285-1342.
Frangogiannis NG, The inflammatory response in myocardial injury, repair, and remodelling. Nat Rev Cardiol. 2014;11(5):255-265.
G. Garrison, S.K. Huang, et al. Reversal of myofibroblast differentiation by prostaglandin e2. Am. J. Respir. Cell Mol. Biol.2013;48:550-558.
Hinz B, Gabbiani G, Fibrosis: recent advances in myofibroblast biology and new therapeutic perspectives. F1000 Biol Rep. 2010;2:78.
Ho JE, Liu C, et al. Galectin-3, a marker of cardiac fibrosis, predicts incident heart failure in the community. J Am Coll Cardiol. 2012 ;60(14):1249-1256.
Jeff M. Berry, Vien Le, et al. Reversibility of Adverse, Calcineurin-Dependent Cardiac Remodeling. Circ Res. 2011;109:407-417.
Kamalov G, Zhao W, et al. Atrophic cardiomyocyte signaling in hypertensive heart disease. J Cardiovasc Pharmacol. 2013;62(6):497-506.
Kapur NK, Wilson S et al. Reduced endoglin activity limits cardiac fibrosis and improves survival in heart failure. Circulation. 2012;125(22):2728-2738.
Karagueuzian, H. S. Targeting cardiac fibrosis: a new frontier in antiarrhythmic therapy? Am. J. Cardiovasc. Dis. 2011;1:101-109.
Kendall RT, Feghali-Bostwick CA, Fibro.blasts in fibrosis: novel roles and mediators. Front Pharmacol. 2014;5:123.
Kong P, Christia P, et al. The pathogenesis of cardiac fibrosis. Cell Mol Life Sci. 2014;71(4):549-574.
Kovacic JC, Mercader N et al. Epithelial-to-mesenchymal and endothelial-to-mesenchymal transition: from cardiovascular development to disease. Circulation. 2012:Apr10;125(14):1795-1808
Li AH, Liu PP, et al. Dynamic changes in myocardial matrix and relevance to disease: translational perspectives. Circ Res. 2014;114(5):916-927.
López, B., Querejeta, R., González, A., Larman, M. & Díez, J. Collagen crosslinking but not collagen amount associates with elevated filling pressures in hypertensive patients with stage C heart failure: potential role of lysyl oxidase. Hypertension 2012; 60:677-683.
Maron BJ, Maron MS, Hypertrophic cardiomyopathy. Lancet. 2013;381(9862):242-255.
Mason D, Chen YZ, et al. Cardiac gene therapy: Recent advances and future directions. J Control Release. 2015;215:101-111.
Mavrogeni S, Markousis-Mavrogenis G, et al. Cardiac involvement in Duchenne and Becker muscular dystrophy. World J Cardiol. 2015;7(7):410-414.
Medici D, Potenta S, and Kalluri R, Transforming growth factor-beta2 promotes Snail-mediated endothelial-mesenchymal transition through convergence of Smad-dependent and Smad-independent signalling. Biochem J 2012;437: 515-520.
Nguyen TP, Qu Z, et al. Cardiac fibrosis and arrhythmogenesis: the road to repair is paved with perils. J Mol Cell Cardiol. 2014;70:83-91.
Oberhaus SM. TUNEL and immunofluorescence double-labeling assay for apoptotic cells with specific antigen(s). Methods Mol Biol 2003;218:85-96.
Okayama K, Azuma J, et al. Hepatocyte growth factor reduces cardiac fibrosis by inhibiting endothelial-mesenchymal transition. Hypertension. 2012;59(5):958-965.
Pedrotty DM, Klinger RY, et al. Cardiac fibroblast paracrine factors alter impulse conduction and ion channel expression of neonatal rat cardiomyocytes. Cardiovasc Res. 2009;83(4):688-697.
Perbal B. CCN proteins: multifunctional signalling regulators. Lancet 2004;363:62-64.
Rincon MY, VandenDriessche T, et al. Gene therapy for cardiovascular disease: advances in vector development, targeting, and delivery for clinical translation. Cardiovasc Res. 2015;108(1):4-20.
Rohr S, Myofibroblasts in diseased hearts: new players in cardiac arrhythmia. Heart Rhythm. 2009;6(6):848-856.
Rosenbloom J, Mendoza FA, et al. Strategies for anti-fibrotic therapies. Biochim Biophys Acta. 2013;1832(7):1088-1103.
Rosin NL, Sopel MJ, et al. Disruption of collagen homeostasis can reverse established age-related myocardial fibrosis. Am J Pathol. 2015;185(3):631-642.
Russo JW, Castellot JJ, et al.CCN5: biology and pathophysiology. J Cell Commun Signal. 2010;4(3):119-130.
Saada JI, Pinchuk IV, Barrera CA, et al. Subepithelial myofibroblasts are novel nonprofessional APCs in the human colonic mucosa. J Immunol 2006;177:5968-5979.
Schelbert EB, Fonarow GC, Therapeutic targets in heart failure: refocusing on the myocardial interstitium. J Am Coll Cardiol. 2014;63(21):2188-2198.
Schroer AK, Merryman WD, et al. Mechanobiology of myofibroblast adhesion in fibrotic cardiac disease. J Cell Sci. 2015 ;128(10):1865-1875.
Skrzypiec-Spring, M, Grotthus, B, Isolated heart perfusion according to Langendorff---still viable in the new millennium. J Pharmacol Toxicol Methods 2007; 55: 113-126.
Sun M, Kisseleva T, Reversibility of liver fibrosis. Clin Res Hepatol Gastroenterol. 2015;39 Suppl 1:560-63.
T. Wynn, Cellular and molecular mechanisms of fibrosis. J. Pathol. 2008:214:199-210.
Van Linthout S, Miteva K, et al. Crosstalk between fibroblasts and inflammatory cells. Cardiovasc Res. 2014;102(2):258-269.
Weber KT, Sun Y, et al. Myofibroblast-mediated mechanisms of pathological remodelling of the heart. Nat Rev Cardiol. 2013;10(1):15-26.
Wei LH, Huang XR, et al. Smad7 inhibits angiotensin II-induced hypertensive cardiac remodelling. Cardiovasc Res. 2013;1;99(4):665-673.
Widyantoro B, Emoto N, Nakayama K, et al. Endothelial cell-derived endothelin-1 promotes cardiac fibrosis in diabetic hearts through stimulation of endothelial-to-mesenchymal transition. Circulation 2010;121:2407-2418.
Wynn TA, Ramalingam TR. Mechanisms of fibrosis: therapeutic translation for fibrotic disease. Nat Med 2012;18:1028-1040.
Yang X, Chen B, et al. Reversal of myofibroblast differentiation: a review. Eur J Pharmacol. 2014;734:83-90.
Zeisberg EM, Tarnavski O, et al. Endothelial-to-mesenchymal transition contributes to cardiac fibrosis. Nat Med 2007;13: 952-961.

## Claims

1. A pharmaceutical composition for use in a method for treating a heart disease associated with Duchenne muscular dystrophy, wherein said composition comprises a CCN5 protein; or a gene carrier including a nucleotide sequence encoding a CCN5 protein, as an active ingredient and wherein said method comprises administering said composition to a subject in need thereof in an amount sufficient to induce myofibroblast-specific apoptosis .

2. The pharmaceutical composition for the use of claim 1, wherein the CCN5 protein includes an amino acid sequence represented by SEQ ID NO: 1.

3. The pharmaceutical composition for the use of claim 1, wherein the nucleotide sequence encoding a CCN5 protein consists of a nucleotide sequence represented by SEQ ID NO: 2.

4. The pharmaceutical composition for the use of claim 1, wherein the gene carrier is selected from the group consisting of a plasmid, an adenovirus, an adeno-associated virus, a retrovirus, a lentivirus, a herpes simplex virus, a vaccinia virus, a liposome, and a niosome.

5. The pharmaceutical composition for the use of claim 1, wherein the gene carrier is an adeno-associated virus.

6. The pharmaceutical composition for the use of claim 5, wherein the adeno-associated virus is selected from the group consisting of adenovirus-associated virus serotype 1, adenovirus-associated virus serotype 6, adenovirus-associated virus serotype 8, and adenovirus-associated virus serotype 9.

## Patentansprüche

1. Pharmazeutische Zusammensetzung zur Verwendung in einem Verfahren zur Behandlung einer Herzerkrankung, die mit Muskeldystrophie des Typs Duchenne verknüpft ist, wobei die Zusammensetzung ein CCN5-Protein oder einen Genträger, der eine Nukleotidsequenz einschließt, die für ein CCN5-Protein kodiert, als aktiven Inhaltsstoff umfasst, und wobei das Verfahren ein Verabreichen der Zusammensetzung an eine darauf angewiesene Person in einer Menge, die ausreicht, um Myofibroblastenspezifische Apoptosis zu induzieren, umfasst.

2. Pharmazeutische Zusammensetzung zur Verwendung nach Anspruch 1, wobei das CCN5-Protein eine Aminosäuresequenz einschließt, die durch SEQ ID NO:1 repräsentiert wird.

3. Pharmazeutische Zusammensetzung zur Verwendung nach Anspruch 1, wobei die Nukleotidsequenz, die für ein CCN5-Protein kodiert, eine Nukleotidsequenz einschließt, die durch SEQ ID NO:2 repräsentiert wird.

4. Pharmazeutische Zusammensetzung zur Verwendung nach Anspruch 1, wobei der Genträger aus der Gruppe ausgewählt wird, die aus einem Plasmid, einem Adenovirus, einem Adeno-assoziierten Virus, einem Retrovirus, einem Lentivirus, einem Herpex-Simplex-Virus, einem Vacciniavirus, einem Liposom und einem Niosom besteht.

5. Pharmazeutische Zusammensetzung zur Verwendung nach Anspruch 1, wobei der Genträger ein Adeno-assoziiertes Virus ist.

6. Pharmazeutische Zusammensetzung zur Verwendung nach Anspruch 5, wobei das Adeno-assozierte Virus aus der Gruppe ausgewählt ist, die aus Adenovirus-assoziiertem Virus vom Serotyp 1, Adenovirus-assoziiertem Virus vom Serotyp 6, Adenovirus-assoziiertem Virus vom Serotyp 8 und Adenovirus-assoziiertem Virus vom Serotyp 9 besteht.

## Revendications

1. Composition pharmaceutique pour une utilisation dans un procédé pour traiter une maladie cardiaque associée à la myopathie de Duchenne, dans laquelle ladite composition comprend une protéine CCN5 ; ou une porteuse de gène incluant une séquence de nucléotides codant pour une protéine CCN5, comme substance active, et dans laquelle ledit procédé comprend l'étape consistant à administrer ladite composition à un sujet en ayant besoin en une quantité suffisante pour induire une apoptose spécifique aux myofibroblastes.

2. Composition pharmaceutique pour l'utilisation selon la revendication 1, dans laquelle la protéine CCN5 inclut une séquence d'acides aminés représentée par la SEQ ID NO : 1.

3. Composition pharmaceutique pour l'utilisation selon la revendication 1, dans laquelle la séquence de nucléotides codant pour une protéine CCN5 consiste en une séquence de nucléotides représentée par la SEQ ID NO : 2.

4. Composition pharmaceutique pour l'utilisation selon la revendication 1, dans laquelle la porteuse de gène est sélectionnée à partir du groupe consistant en un plasmide, un adénovirus, un virus adéno-associé, un rétrovirus, un lentivirus, un virus de l'herpès simplex, un virus de la vaccine, un liposome et un niosome.

5. Composition pharmaceutique pour l'utilisation selon la revendication 1, dans laquelle la porteuse de gène est un virus adéno-associé.

6. Composition pharmaceutique pour l'utilisation selon la revendication 5, dans laquelle le virus adéno-associé est sélectionné à partir du groupe consistant en un virus adéno-associé de sérotype 1, un virus adéno-associé de sérotype 6, un virus adéno-associé de sérotype 8 et un virus adéno-associé de sérotype 9.
